# EUROPEAN PATENT APPLICATION

(11) **EP 4 617 280 A1**
(43) Date of publication of application: **17.09.2025**
(21) Application number: 23887658.5
(22) Date of filing: 18.09.2023
(51) Int. Cl.: C07H 19/207, C07H 1/02, C07H 1/04, C07H 1/06, C12P 19/34

(54) **VINYLPHOSPHONIC ACID-MODIFIED MRNA CAP ANALOGUE, AND PREPARATION METHOD THEREFOR AND USE THEREOF**

(30) Priority: 08.11.2022 CN 202211395011
(71) Applicant: Jiangsu Synthgene Biotechnology Co., Ltd., Nanjing, Jiangsu 210000 (CN)
(72) Inventor: HUANG, Lei, Nanjing, Jiangsu 210000 (CN); ZHAO, Wannian, Nanjing, Jiangsu 210000 (CN); SHEN, Qi, Nanjing, Jiangsu 210000 (CN)
(74) Representative: Mewburn Ellis LLP
(86) International application number: PCT/CN2023/119496
(87) International publication number: WO 2024/098964

(57) **Abstract**

The present invention provides a vinylphosphonic acid-modified mRNA cap analog, a method for preparing same, and use thereof. The vinylphosphonic acid-modified mRNA cap analog of the present invention has a more stable molecular conformation due to the existence of a double bond structure, thereby being less susceptible to recognition and hydrolysis by a nuclease, and also shows good *in-vitro* transcription yield and capping efficiency data. Meanwhile, the mRNA cap analog containing the double bond has better binding ability to a eukaryotic initiation factor (elF4E) and shows better mRNA translation effect.

## Description

The present application claims priority to the Chinese Patent Application No. 202211395011.5 entitled "VINYLPHOSPHONIC ACID-MODIFIED MRNA CAP ANALOG, METHOD FOR PREPARING SAME, AND USE THEREOF" and filed with the China National Intellectual Property Administration on November 08, 2022, which is incorporated herein by reference in its entirety.

### TECHNICAL FIELD

The present invention relates to the technical field of chemical and biological engineering, and particularly to a vinylphosphonic acid-modified mRNA cap analog, a method for preparing same, and use thereof.

### BACKGROUND

Eukaryotic mRNAs bear a "cap" structure at their 5'-ends, which is well known to play an important role in translation. A naturally occurring cap structure consists of a 7-methylguanosine that is linked via a triphosphate bridge to the 5'-end of the first transcribed nucleotide, resulting in 7G(5')ppp(5')N, where N is any nucleotide. The mRNA cap plays an important role in gene expression. The cap structure protects the mRNAs from degradation by exonucleases, enables the transport of RNAs from the nucleus to the cytoplasm, and participates in the assembly of a translation initiation complex. m7G(5')ppp(5')G (mCAP) has been used as a primer for transcription with T7 or SP6 RNA polymerase *in vitro* to obtain RNAs having a cap structure at their 5'-ends.

Synthesis of mRNAs by *in-vitro* transcription has become an important tool for introducing foreign genes and expressing proteins, is widely used in the treatment and prevention of diseases, and allows workers to prepare RNA molecules that perform appropriately in various biological applications. Such applications include research applications and commercial production of polypeptides, for example, production in cell-free translation systems of polypeptides containing "unnatural" amino acids at specific sites, or production in cultured cells of polypeptides that require post-translational modification for their activity or stability. In the latter system, synthesis takes significantly longer, and therefore, more proteins are produced.

Based on the structural characteristics, in the prior art, the level of the binding ability of the 5'-end cap structure of mRNA to a eukaryotic initiation factor (elF4E) determines the translation and expression effect of the mRNA in cells. Existing cap analog structures are generally natural cap structures, which have unstable mRNA translation effects that vary greatly for different sequences or different expression hosts.

### SUMMARY

The present application provides a vinylphosphonic acid-modified mRNA cap analog, a method for preparing same, and use thereof. Vinylphosphonic acid is used for simulating normal phosphonic acid function in the structure of the mRNA cap analog, and due to the structural improvement, the vinylphosphonic acid-modified mRNA cap analog has the advantage of spatial conformation and exhibits better binding ability with the eukaryotic initiation factor (elF4E), which can ultimately improve the translation level of mRNA in cells.

Provided is a vinylphosphonic acid-modified mRNA cap analog, comprising the following structure, or a pharmaceutically acceptable salt or stereoisomer thereof: 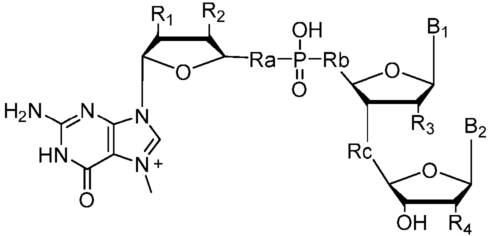
wherein R₁, R₂, R₃, and R₄ are independently hydrogen, halogen, hydroxyl, substituted or unsubstituted alkyl, substituted or unsubstituted O-alkyl, substituted or unsubstituted S-alkyl, substituted or unsubstituted NH-alkyl, substituted or unsubstituted N-dihydrocarbyl, substituted or unsubstituted O-aryl, substituted or unsubstituted S-aryl, substituted or unsubstituted NH-aryl, substituted or unsubstituted O-aralkyl, substituted or unsubstituted S-aralkyl, or substituted or unsubstituted NH-aralkyl;
B₁ and B₂ are independently a natural, modified, or non-natural nucleobase;
Ra, Rb, and Rc independently have the following structure: 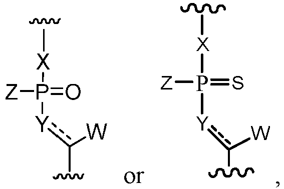 wherein Y is O, OCH₂, OCH, CH₂, CH, C₂H₃, or C₃H₅; Z is OH, SH, BH₃, aryl, alkyl, O-alkyl, or O-aryl; X is O, CH₂, or NH; W is H, OH, alkyl, O-alkyl, N-alkyl, S-alkyl, or halogen; -̅ -̅ -̅ represents a double bond or a single bond and is connected to a five-membered sugar ring;
and in at least one of the structures Ra, Rb and Rc, Y is O, OCH, or CH.

The "comprising" described above may be replaced by "having".

R₁, R₂, R₃, and R₄ described above are independently hydrogen, halogen, hydroxyl, substituted or unsubstituted alkyl, substituted or unsubstituted O-alkyl, substituted or unsubstituted S-alkyl, or substituted or unsubstituted NH-alkyl.

The halogen described above is F, Cl, or Br.

The number of carbon atoms in the substituted or unsubstituted alkyl described above is 0-5, preferably 1-5;
preferably, the number of carbon atoms in the substituted or unsubstituted alkyl described above is 1, 2, or 3.

Preferably, the substituted or unsubstituted alkyl described above is substituted or unsubstituted methyl, substituted or unsubstituted ethyl, substituted or unsubstituted n-propyl, substituted or unsubstituted isopropyl, substituted or unsubstituted n-butyl, substituted or unsubstituted isobutyl, or substituted or unsubstituted tert-butyl.

The number of carbon atoms in the substituted or unsubstituted O-alkyl described above is 0-5, preferably 1-5;
preferably, the number of carbon atoms in the substituted or unsubstituted O-alkyl described above is 1, 2, or 3.

Preferably, the substituted or unsubstituted O-alkyl described above is substituted or unsubstituted methoxy, substituted or unsubstituted ethoxy, or substituted or unsubstituted propoxy.

The number of carbon atoms in the substituted or unsubstituted S-alkyl described above is 0-5, preferably 1-5;
Preferably, the number of carbon atoms in the substituted or unsubstituted S-alkyl described above is 1, 2, or 3.

Preferably, the substituted or unsubstituted S-alkyl described above is substituted or unsubstituted methylthio, substituted or unsubstituted ethylthio, or substituted or unsubstituted propylthio.

The number of carbon atoms in the substituted or unsubstituted NH-alkyl described above is 0-5, preferably 1-5;
preferably, the number of carbon atoms in the substituted or unsubstituted NH-alkyl described above is 1, 2, 3, 4, or 5.

Preferably, the substituted or unsubstituted NH-alkyl described above is substituted or unsubstituted methylamino, or substituted or unsubstituted ethylamino or propylamino.

Preferably, the number of carbon atoms in the substituted or unsubstituted N-dihydrocarbyl described above is 0-5, preferably 1-5.

Substituent groups in the alkyl, O-alkyl, S-alkyl, NH-alkyl and N-dihydrocarbyl described above are independently selected from one or more of substituted or unsubstituted alkyl, substituted or unsubstituted O-alkyl, and substituted or unsubstituted acylamino.

Preferably, the number of carbon atoms in the alkyl described above is 1-5; the number of carbon atoms in the O-alkyl described above is 1-5; and the number of carbon atoms in the acylamino described above is 1-5. Preferably, the alkyl, O-alkyl and acylamino described above may be further substituted with C1-C5 linear or branched alkyl. Preferably, the hydrogen connected to the N atom in the acylamino described above may be substituted with C1-C5 linear or branched alkyl. Preferably, the hydrogen connected to the N atom in the acylamino described above may be substituted with one or more of methyl, ethyl, *n*-propyl, isopropyl, *n*-butyl, isobutyl, *tert*-butyl, *n*-pentyl, and isopentyl.

Preferably, the substituent groups in the alkyl, O-alkyl, S-alkyl, NH-alkyl and N-dihydrocarbyl described above are independently selected from methyl, ethyl, *n-*propyl, isopropyl, methoxy, ethoxy, *n*-propoxy, isopropoxy, formamido, acetamido, *n-*propylamido, isopropylamido, *n*-butyramido, isobutyramido, *tert*-butyramido, *N-*methylacetamido, *N*-ethylacetamido, *N-n*-propylacetamido, and *N-*isopropylacetamido.

Preferably, R₁, R₂, R₃, and R₄ described above are independently hydrogen, F, hydroxyl, methoxy, ethoxy, propoxy, methoxymethyl, methoxyethoxy, acetamidomethyl, *n*-propylamidomethyl, isopropylamidomethyl, butyramidomethyl, *N*-methylacetamidomethyl, *N*-ethylacetamidomethyl, *N-n*-propylacetamidomethyl, or *N*-isopropylacetamidomethyl.

B₁ and B₂ described above are independently adenine, guanine, cytosine, uracil, or thymine.

Ra, Rb, and Rc described above independently have the following structure: 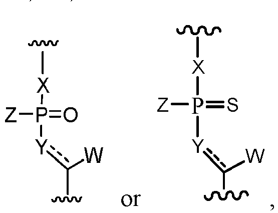 wherein Y is O, CH, C₂H₃, or C₃H₅; Z is OH or alkyl; X is O or CH₂; and W is H, OH, or alkyl;
the number of carbon atoms in the alkyl described above is 0-5.

The number of carbon atoms in the alkyl described above is 1, 2, or 3.

In at least one of the structures Ra, Rb and Rc, Y is CH.

R₁, R₂, and R₄ described above are all hydroxyl, and R₃ is methoxy; B₁ is adenine, and B₂ is guanine;
one or two of Ra, Rb, and Rc have the following structure: 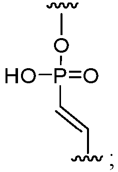
optionally, the vinylphosphonic acid-modified mRNA cap analog described above has any one of the following structures, or a pharmaceutically acceptable salt, stereoisomer or the like thereof: 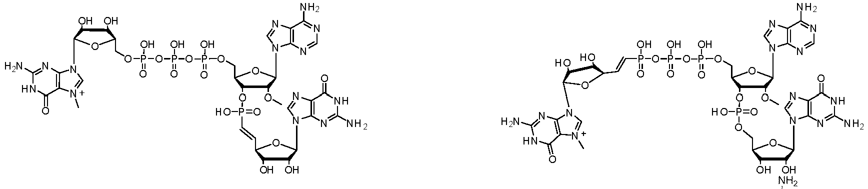 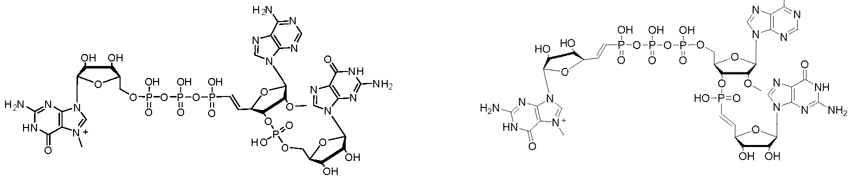 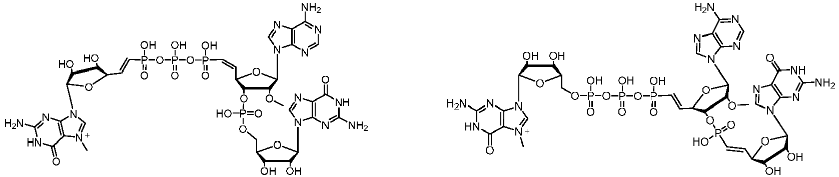 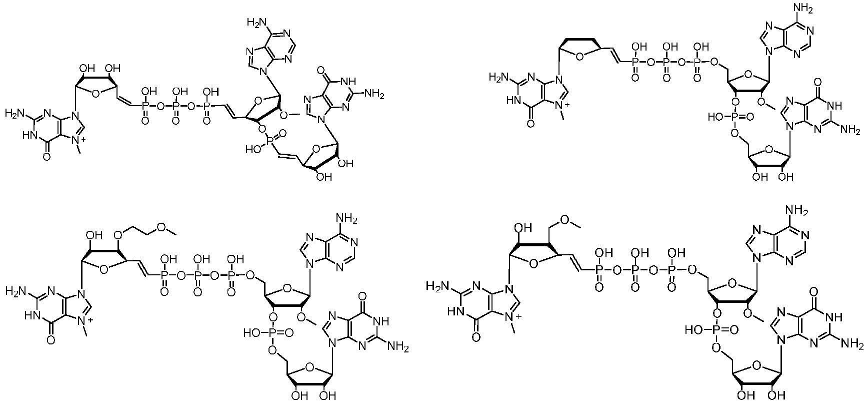 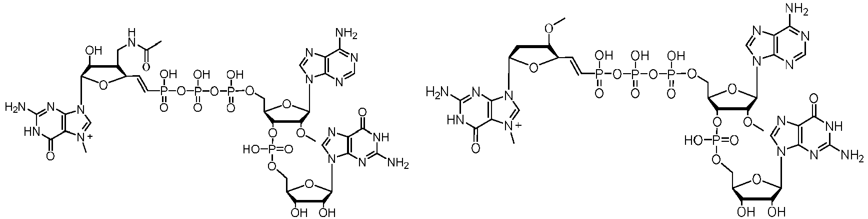 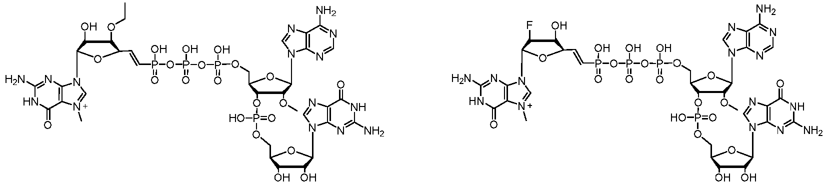 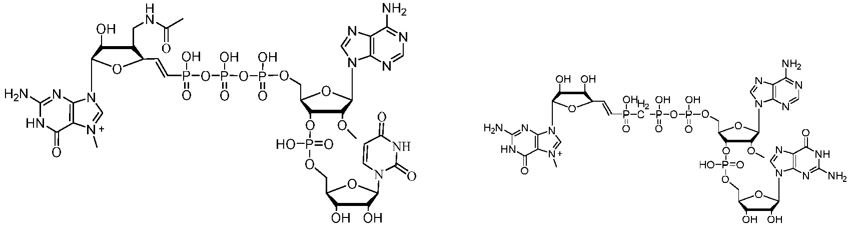 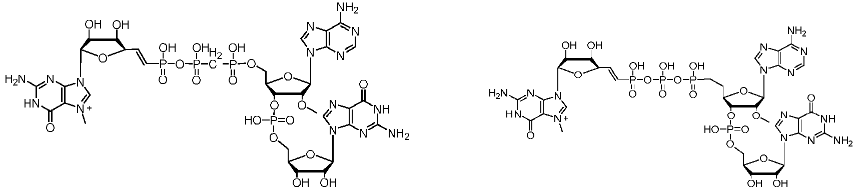 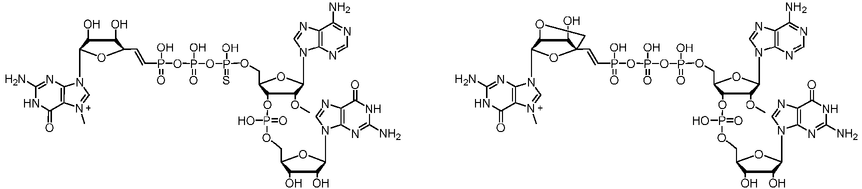 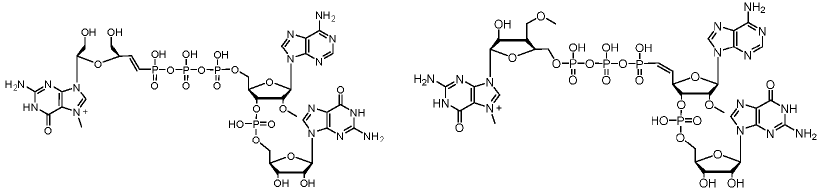 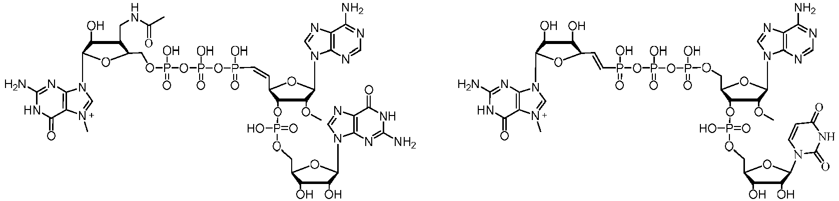 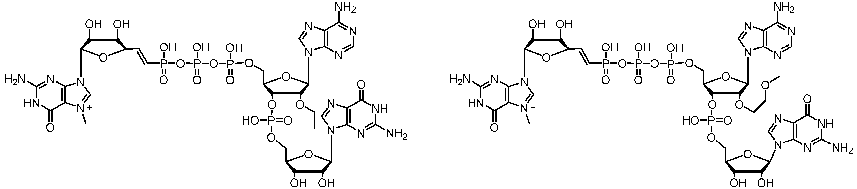 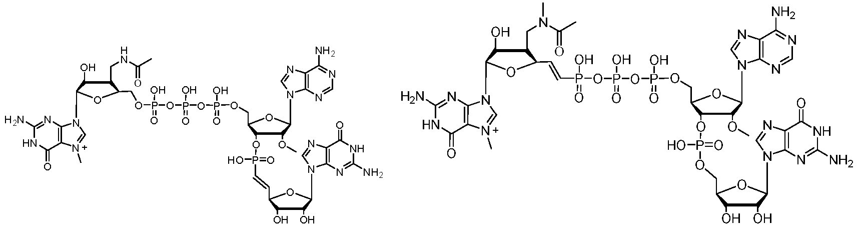 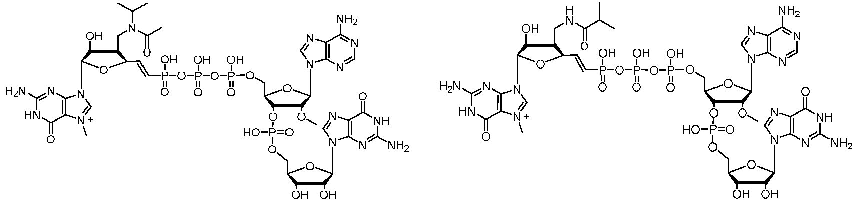 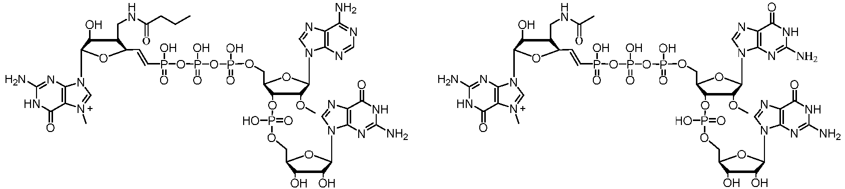
preferably, only one of Ra, Rb, and Rc described above has the following structure:

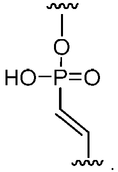

The vinylphosphonic acid-modified mRNA cap analog provided herein may also be in the form of a salt, wherein the salt may be a salt well known to those skilled in the art such as a sodium salt, a potassium salt, an ammonium salt, or an organic amine salt.

The cation of the ammonium salt is NH₄⁺.

The organic amine salt includes, but is not limited to, triethylamine salt.

The vinylphosphonic acid-modified mRNA cap analog provided herein may also be a stereoisomer of the structure described above. The "stereoisomer" refers to compounds having the same chemical structure but differing in the arrangement of atoms or groups in space. The stereoisomer described above includes, but is not limited to an enantiomer, a diastereoisomer, a conformational isomer (rotamer), a geometric isomer (cis/trans isomer), an atropisomer, and the like.

In an alternative embodiment, the vinylphosphonic acid-modified mRNA cap analog described above has a carbon-carbon double bond of at least one of form E or form Z.

Provided is a method for preparing the vinylphosphonic acid-modified mRNA cap analog, comprising the following steps: (1) synthesis of an imidazole salt of m7GDP or a modified analog thereof; (2) preparation of a phosphoester bond-linked dinucleotide; and (3) synthesis of the vinylphosphonic acid-modified mRNA cap analog.

The method specifically comprises the following steps: (1) the synthesis of the imidazole salt of m7GDP or the modified analog thereof: sequentially subjecting a guanosine or an analog thereof to diphosphorylation, methylation of N7, imidazolation reaction of diphosphoric acid, and the like to synthesize the imidazole salt of m7GDP or the modified analog thereof; (2) the preparation of the phosphoester bond-linked dinucleotide: coupling 2'OMe-A or a modified phosphoramidite monomer thereof with a protected guanosine or a modified analog thereof under the action of tetrazole to form a first phosphoester bond, removing a protecting group under the action of an acid, then introducing a second phosphoric acid, and finally performing hydrolysis to obtain the phosphoester bond-linked dinucleotide; and (3) the synthesis of the vinylphosphonic acid-modified mRNA cap analog: reacting the imidazole salt of m7GDP or the modified analog thereof with the phosphoester bond-linked dinucleotide to prepare the vinylphosphonic acid-modified mRNA cap analog.

The method comprises the following specific steps:

### (1) The synthesis of the imidazole salt of m7GDP or the modified analog thereof:

1) Guanosine or an analog thereof is weighed and dissolved in trimethyl phosphate, and the reaction solution is cooled to 0 °C. Phosphorus oxychloride (1.8 eq.) is slowly added dropwise, and after the mixture is stirred for 4 h under an ice bath, water is added to quench the reaction. The mixture is then purified by reverse-phase chromatography to obtain an intermediate a. 2) The intermediate a, triphenylphosphine (1.0 eq.), 2,2'-dithiodipyridine (2.0 eq.), imidazole (8.0 eq.), and triethylamine (1.0 eq.) are dissolved in DMF, and the reaction solution is well stirred for 10 h. After the reaction is completed, a 4 M solution of sodium perchlorate in acetone is added for precipitation. The mixture is filtered, and the filter cake is thoroughly washed with acetone to obtain an intermediate b. 3) The intermediate b is dissolved in DMF, and tributylamine phosphate (3.0 eq.) and zinc chloride (8.0 eq.) are added. The mixture is then stirred at room temperature for 5 h. After the reaction is completed, the mixture is purified by ion chromatography to obtain an intermediate c. 4) The intermediate c is dissolved in 20 volumes of purified water, and the reaction solution is cooled to 4 °C. Dimethyl sulfate is then slowly added dropwise, and the pH is adjusted to no more than 5 with 2 M sodium hydroxide in the process. After the reaction is completed as detected by HPLC, the mixture is purified by ion chromatography to obtain an intermediate d. 5) The intermediate d, triphenylphosphine (1.0 eq.), 2,2'-dithiodipyridine (2.0 eq.), imidazole (8.0 eq.), and triethylamine (1.0 eq.) are dissolved in DMF, and the reaction solution is well stirred for 10 h. After the reaction is completed, a 4 M solution of sodium perchlorate in acetone is added for precipitation. The mixture is filtered, and the filter cake is thoroughly washed with acetone to obtain the imidazole salt of m7GDP or the modified analog thereof.

### (2) The preparation of the phosphoester bond-linked dinucleotide:

2'OMe-rA or a modified phosphoramidite monomer thereof is weighed into a single-necked flask and dissolved in dichloromethane, and then 2',3'-acetylguanosine or an analog thereof is added. The mixture is cooled to 25±2 °C, and tetrazole is added under nitrogen purge. The mixture is then heated to 25±2 °C for reaction. After the completion of the reaction is detected, an I₂/pyridine solution (1.2 eq.) is added to the reaction solution. After the completion of the reaction is detected, the mixture is concentrated by rotary evaporation. The concentrated ointment is dissolved in dichloromethane, and trifluoroacetic acid (1.1 eq.) is added. After the completion of the reaction is detected, the mixture is concentrated by rotary evaporation, slurried with petroleum ether/dichloromethane in a certain ratio, and filtered to obtain an intermediate e. The intermediate e is dissolved in acetonitrile, and a phosphine reagent (1.2 eq.) and tetrazole (1.2 eq.) are added. The mixture is well stirred for reaction. After the completion of the reaction is detected, an I₂/pyridine solution (1.2 eq.) is further added to the reaction solution. After the completion of the reaction is detected, the mixture is concentrated by rotary evaporation. 3 L of methanol and 3 L of concentrated aqueous ammonia are added into the rotary flask, and the mixture is allowed to react at room temperature for 4 h. After the completion of the reaction is detected, the mixture is concentrated by rotary evaporation. 20 L of ultrapure water is added, and the mixture is loaded into a reverse ion permeation device, washed, concentrated, and lyophilized to obtain the phosphoester bond-linked dinucleotide.

### (3) The synthesis of the vinylphosphonic acid-modified mRNA cap analog:

The imidazole salt of m7GDP or the modified analog thereof obtained in step (1) is dissolved in a DMF solution containing MnCl₂, and the resulting solution is added to a solution of the phosphoester bond-linked dinucleotide obtained in step (2) in DMF. The mixture is stirred at room temperature for reaction, and after 24 h, the reaction is terminated with a 0.25 M EDTA solution, and the mixture is loaded onto a DEAESephadex column. The product is eluted with a linear gradient using a 0-1.0 M TEAB eluent. An elution product with an HPLC purity of more than 97% is collected, and the separation solution is concentrated, then loaded onto a strong anion resin, and eluted with a linear gradient using a 0-1.0 M sodium acetate eluent. An elution product with an HPLC purity of more than 98.5% is collected. The high-purity eluates are combined, filtered through a nanofiltration device to remove the residual sodium acetate solution, and then concentrated to obtain the target product, i.e., the vinylphosphonic acid-modified mRNA cap analog.

Provided is use of the vinylphosphonic acid-modified mRNA cap analog, wherein the vinylphosphonic acid-modified mRNA cap analog is used for mRNA capping in a T7 RNA polymerase system. T7 RNA polymerase is a DNA-dependent RNA polymerase that has high specificity for phage T7 promoter sequences. This enzyme synthesizes large quantities of RNA from DNA inserted into a transcription vector downstream from a T7 promoter. The T7 RNA polymerase-catalyzed IVT (*in vitro* transcription) reaction system is currently the most mature system for mRNA preparation.

Typically, 20 µL of the IVT reaction system containing 50 U of the T7 RNA polymerase along with 1 µL of the cap analog (100 mM) can achieve optimal transcription yield and capping efficiency.

The present invention provides a complex, comprising the vinylphosphonic acid-modified mRNA cap analog described above and a DNA template, wherein the DNA template comprises a promoter region comprising a transcription start site, the transcription start site having a first nucleotide at nucleotide position +1 and a second nucleotide at nucleotide position +2; the group B₁ in the structure of the vinylphosphonic acid-modified mRNA cap analog is complementary to a base of the first nucleotide, and the group B₂ in the structure of the vinylphosphonic acid-modified mRNA cap analog is complementary to a base of the second nucleotide.

The present invention provides an RNA molecule, comprising the vinylphosphonic acid-modified mRNA cap analog described above. The vinylphosphonic acid-modified mRNA cap analog described above serves as a cap structure or cap structure fragment of the RNA molecule described above.

The present invention provides a pharmaceutical composition, comprising the RNA molecule described above and a pharmaceutically acceptable carrier.

According to the vinylphosphonic acid-modified mRNA cap analog, the method for preparing same, and the use thereof provided herein, the vinylphosphonic acid-modified mRNA cap analog is suitable for mRNA produced by utilizing an *in-vitro* co-transcription method using a DNA sequence as a template, wherein the DNA sequence can be derived or modified from viruses, animals, plants, and other species, and meanwhile, the produced mRNA has lower immunogenicity, higher protein translation efficiency, and better stability.

Compared with the prior art, the present invention has the following advantages:
1. Compared with the existing cap structure analog Cleancap, the vinylphosphonic acid-modified mRNA cap analog of the present invention has a more stable molecular conformation due to the existence of a double bond structure, thereby being less susceptible to recognition and hydrolysis by a nuclease, and also shows better *in-vitro* transcription yield and capping efficiency data. Meanwhile, the mRNA cap analog containing the double bond has better binding ability to the eukaryotic initiation factor (elF4E) and shows better mRNA translation effect.
2. The vinylphosphonic acid-modified cap structure designed by the present invention comprises a vinyl group, which can effectively fix the spatial structure of the 5'-end cap and improve the binding ability of the 5'-end cap structure of mRNA to the eukaryotic initiation factor (elF4E), thereby improving the translation effect of mRNA.

### DETAILED DESCRIPTION

The technical solutions of the present invention will be clearly and completely described below with reference to the examples of the present invention, and it is obvious that the described examples are only a part of the examples of the present invention but not all of them. Based on the embodiments of the present invention, all other embodiments derived by those of ordinary skill in the art without creative work shall fall within the protection scope of the present invention.

Terminology definition: In the present invention, "eq." means equivalent.

The names and sources of the starting materials used in the examples are shown in Table 1 below:

**Table 1**

| Reagent | Purchasing manufacturer |
|---|---|
| MnCl₂ | Shanghai Aladdin Biochemical Technology Co., Ltd. |
| Guanosine | Anhui Senrise Technology Co., Ltd. |
| Trimethyl phosphate | Anhui Senrise Technology Co., Ltd. |
| Phosphorus oxychloride | Anhui Senrise Technology Co., Ltd. |
| Zinc chloride | Anhui Senrise Technology Co., Ltd. |
| Imidazole | Anhui Senrise Technology Co., Ltd. |
| Tetrazole | Sinopharm Chemical Reagent Co., Ltd. |
| Triphenylphosphine | Anhui Senrise Technology Co., Ltd. |
| Trimethylchlorosilane | Anhui Senrise Technology Co., Ltd. |
| Isobutyryl chloride | Anhui Senrise Technology Co., Ltd. |
| 4,4'-Dimethoxytrityl chloride | Anhui Senrise Technology Co., Ltd. |
| Tetraethyl methylenediphosphonate | Shanghai Macklin Biochemical Co., Ltd. |
| Bis(2-cy anoethyl)-N,N-diisopropylphosphoramidite | Xinxiang Runyu Material Co., Ltd. |
| 2'OMe-rA phosphoramidite monomer | Wuhu Huaren Science and Technology Co., Ltd. |
| NaH | Anhui Senrise Technology Co., Ltd. |
| Triethylbromosilane | Anhui Senrise Technology Co., Ltd. |
| 2,2'-Dithiodipyridine | Shanghai Macklin Biochemical Co., Ltd. |
| Dimethyl sulfate | Anhui Senrise Technology Co., Ltd. |
| EDCI | Anhui Senrise Technology Co., Ltd. |
| DMSO | Anhui Senrise Technology Co., Ltd. |
| DMF | Anhui Senrise Technology Co., Ltd. |

### Example 1: Synthesis method for vinylphosphonic acid-modified mRNA cap analog using intermediates A and B as starting materials

Intermediate A (2 mol) was dissolved in a DMF solution containing MnCl₂ (20 mol), and then intermediate B (1.8 mol) was added to the reaction solution. The mixture was stirred at room temperature for reaction. After 24 h, the reaction was terminated with 10 L of a 0.25 M EDTA solution. The mixture was loaded onto a DEAE Sephadex column (30 × 500 cm). The product was eluted with a linear gradient using a 0-1.0 M TEAB eluent. The eluate was concentrated to obtain the target product. The reaction route is shown in equation (1):

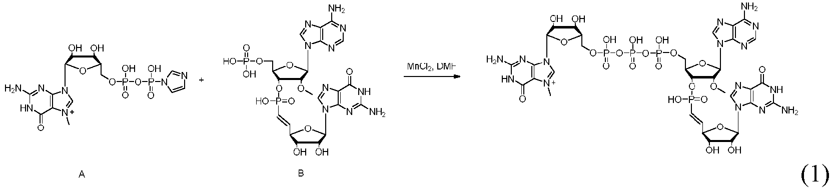

In the route, compound A was obtained by the following steps: 1) 5 g of guanosine was weighed and dissolved in 70.0 mL of trimethyl phosphate, and the reaction solution was cooled to 0 °C. Phosphorus oxychloride (1.8 eq.) was slowly added dropwise, and after the mixture was stirred for 4 h under an ice bath, water was added to quench the reaction. The mixture was then purified by reverse-phase chromatography to obtain intermediate A1. 2) Intermediate A1, triphenylphosphine (1.0 eq.), 2,2'-dithiodipyridine (2.0 eq.), imidazole (8.0 eq.), and triethylamine (1.0 eq.) were dissolved in DMF, and the reaction solution was well stirred for 10 h. After the reaction was completed, a 4 M solution of sodium perchlorate in acetone was added for precipitation. The mixture was filtered, and the filter cake was thoroughly washed with acetone to obtain intermediate A2. 3) 10 g of intermediate A2 was dissolved in DMF, and tributylamine phosphate (3.0 eq.) and zinc chloride (8.0 eq.) were added. The mixture was then stirred at room temperature for 5 h. After the reaction was completed, the mixture was purified by ion chromatography to obtain intermediate A3. 4) Intermediate A3 was dissolved in 20 volumes of purified water, and the reaction solution was cooled to 4 °C. Dimethyl sulfate was then slowly added dropwise, and the pH was adjusted to no more than 5 with 2 M sodium hydroxide in the process. After the reaction was completed as detected by HPLC, the mixture was purified by ion chromatography to obtain intermediate A4. 5) Intermediate A4, triphenylphosphine (1.0 eq.), 2,2'-dithiodipyridine (2.0 eq.), imidazole (8.0 eq.), and triethylamine (1.0 eq.) were dissolved in DMF, and the reaction solution was well stirred for 10 h. After the reaction was completed, a 4 M solution of sodium perchlorate in acetone was added for precipitation. The mixture was filtered, and the filter cake was thoroughly washed with acetone to obtain intermediate A.

The reaction route for compound A is shown in equation (2):

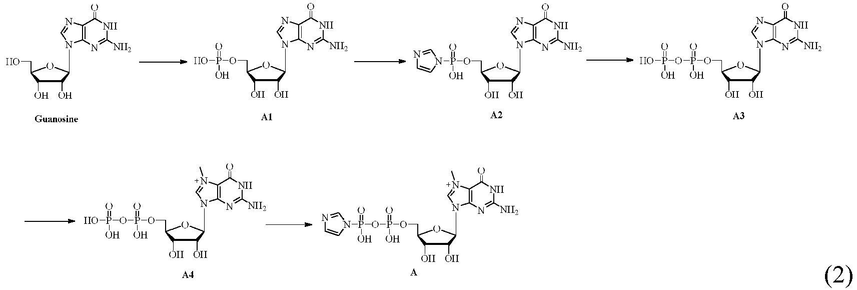

In the route, compound B was obtained by the following steps: 1) Guanosine (10 g) was dissolved in 150 mL of pyridine, and trimethylchlorosilane (5.0 eq.) was added to the reaction solution. The mixture was then stirred at room temperature for 2 h and cooled to 0 °C. Isobutyryl chloride (1.5 eq.) was added dropwise to the reaction solution, and then the mixture was allowed to react at room temperature for 3 h. 20 mL of aqueous ammonia and 500 mL of water were sequentially added dropwise to the reaction solution under an ice bath, and the aqueous phase was washed with dichloromethane and then recrystallized with hot water to obtain intermediate B1.
2) 10 g of intermediate B1 was dissolved in 150 mL of pyridine, and the reaction solution was cooled to 0 °C. A solution of 4,4'-dimethoxytrityl chloride (1.2 eq.) in pyridine (50 mL) was then added dropwise to the reaction solution. The mixture was stirred at room temperature for 4 h until the reaction was completed as detected by TLC. The reaction was quenched with a 5% aqueous NaHCO₃ solution under an ice bath. The mixture was extracted with ethyl acetate and then crystallized with *n*-hexane to obtain intermediate B2.
3) 10 g of intermediate B2 was dissolved in 100 mL of a DMF solution, and TBDMSCl (3.1 eq.) was added to the reaction solution. The mixture was stirred at room temperature overnight. A saturated aqueous sodium carbonate solution was added, and the mixture was extracted with ethyl acetate and then crystallized to obtain intermediate B3.
4) 8 g of intermediate B3 was dissolved in 80 mL of an 80% acetic acid solution, and the solution was stirred at room temperature for 5 h. 400 mL of ethyl acetate was added, and then the pH was adjusted to neutrality with saturated sodium carbonate. The organic phase was separated, concentrated, and then subjected to column chromatography to obtain intermediate B4.
5) 5 g of intermediate B4 was dissolved in 50 mL of DMF, and DMSO (6.0 eq.) and EDCI (3.0 eq.) were added to the reaction solution before pyridine (1.0 eq.) and trifluoroacetic acid (1.0 eq.) were added dropwise. The mixture was allowed to react at room temperature for 5 h, and then the reaction solution was diluted with ethyl acetate. The organic phase was separately washed with a saturated aqueous sodium bicarbonate solution and water, then concentrated, and subjected to column chromatography to obtain intermediate B5.
6) Tetraethyl methylenediphosphonate (1.5 eq.) was added dropwise to a suspension of NaH (60%, 1.0 eq.) in THF (80 mL) under an ice bath, and then the mixture was stirred at 0 °C for 0.5 h. A solution of intermediate B5 (3 g) in THF (40 mL) was slowly added dropwise to the reaction solution. The mixture was stirred at room temperature overnight. A saturated aqueous ammonium chloride solution was added dropwise to the reaction solution under an ice bath to quench the reaction, and the mixture was extracted with ethyl acetate, then concentrated, and subjected to column chromatography to obtain intermediate B6.
7) 2 g of intermediate B6 was dissolved in 40 mL of acetonitrile, and triethylbromosilane (10.0 eq.) was added to the reaction solution. The mixture was heated to 75 °C, incubated overnight, and then concentrated. The crude product was subjected to reverse-phase preparative chromatography to obtain intermediate B7.
8) Intermediate B7 (1 g) and DMT-2'OMe-A(Bz) (1.2 eq.) were dissolved in DMF, and DCC (2.5 eq.) was added to the reaction solution. The mixture was incubated at room temperature overnight, concentrated, and then subjected to column chromatography to obtain intermediate B8.
9) 1 g of intermediate B8 was dissolved in an 80% aqueous acetic acid solution, and the solution was stirred at room temperature overnight. After the reaction was completed, the reaction solution was concentrated and then purified to obtain intermediate B9.
10) 0.6 g of intermediate B9 was dissolved in dichloromethane (12 mL), and tetrazole (1.1 eq.) and bis(2-cyanoethyl)-*N,N*-diisopropylphosphoramidite (1.1 eq.) were separately added to the reaction solution. The mixture was allowed to react at room temperature for 5 h, and after the reaction was completed, the mixture was concentrated to obtain a crude product of intermediate B10.
11) The crude product of intermediate B10 was dissolved in 20 mL of aqueous ammonia, and the solution was heated to 50 °C and incubated overnight. After the reaction was completed, the reaction solution was concentrated and purified by reverse-phase chromatography to obtain intermediate B.

The reaction route for compound B is shown in equation (3):

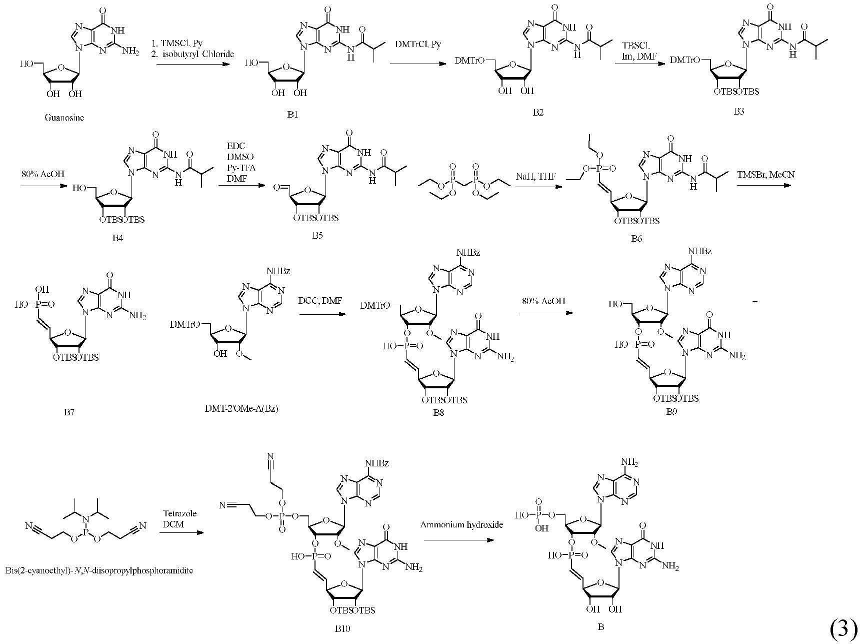

### Example 2: Synthesis method for vinylphosphonic acid-modified mRNA cap analog using intermediates C and D as starting materials

With reference to the synthesis method for the target product of Example 1, the vinylphosphonic acid-modified mRNA cap analog of Example 2 was obtained using intermediates C and D as starting materials. The reaction route is shown in equation (4):

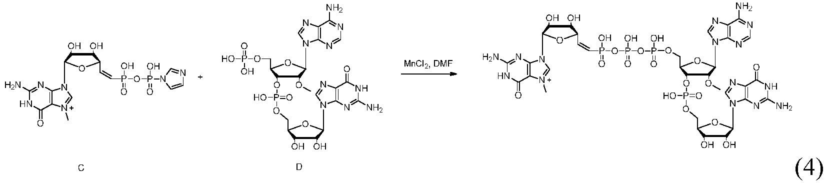

In the route, compound C was obtained by the following steps: 1) 5 g of intermediate B7 was dissolved in H₂O/TFA (1:1, 50 mL), and the solution was stirred at room temperature for 1 h. After the reaction was completed, the solution was purified by reverse-phase chromatography to obtain intermediate C1.
2) Intermediate C1 (3 g), triphenylphosphine (1.5 eq.), 2,2'-dithiodipyridine (1.5 eq.), triethylamine (2.5 eq.), and imidazole (8.0 eq.) were dissolved in 20.0 mL of DMF, and the solution was stirred at room temperature for 10 h. After the reaction was completed, a 4 M solution of sodium perchlorate in acetone was added to the reaction solution for precipitation. The mixture was filtered, and the filter cake was thoroughly washed with acetone to obtain intermediate C2.
3) 2 g of intermediate C2 was weighed and dissolved in DMF, and triethylamine phosphate (3.0 eq.) and zinc chloride (8.0 eq.) were added. The mixture was stirred at room temperature for 8 h. After the reaction was completed, the mixture was purified by reverse-phase chromatography to obtain intermediate C3.
4) 2 g of intermediate C3 was dissolved in 40 mL of purified water, and the reaction solution was cooled to 4 °C. Dimethyl sulfate was then slowly added dropwise, and the pH was adjusted to no more than 5 with 2 M sodium hydroxide in the process. After the reaction was completed as detected by HPLC, the mixture was purified by ion chromatography to obtain intermediate C4.
5) With reference to the synthesis method for intermediate C2, intermediate C was obtained.

The reaction route for compound C is shown in equation (5):

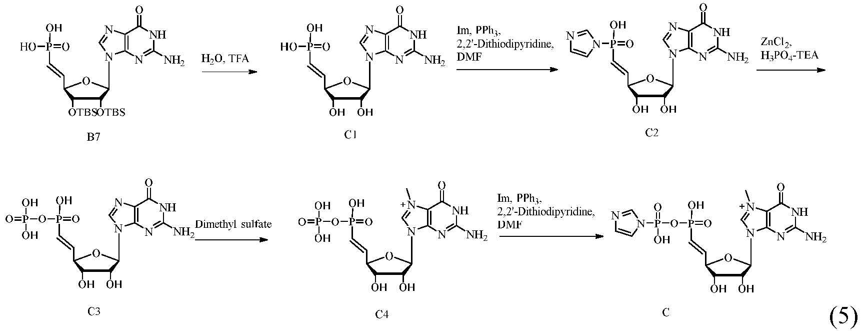

In the route, compound D was obtained by the following steps: 5 kg of 2'OMe-rA phosphoramidite monomer was weighed into a single-necked flask and dissolved in 50 L of dichloromethane, and then 2.73 kg of 2',3'-acetylguanosine was added. The mixture was cooled to 25±2 °C, and 880 g of tetrazole was added under nitrogen purge. The mixture was then heated to 25±2 °C for reaction. After the completion of the reaction was detected, an I₂/pyridine solution (1.2 eq.) was added to the reaction solution. After the completion of the reaction was detected, the mixture was concentrated by rotary evaporation. The concentrated ointment was dissolved in 4 L of dichloromethane, and trifluoroacetic acid (1.1 eq.) was added. After the completion of the reaction was detected, the mixture was concentrated by rotary evaporation, slurried with petroleum ether/dichloromethane in a certain ratio, and filtered to obtain intermediate D1. The intermediate D1 was dissolved in 4 L of acetonitrile, and a phosphine reagent (1.2 eq.) and tetrazole (1.2 eq.) were added. The mixture was well stirred for reaction. After the completion of the reaction was detected, an I₂/pyridine solution (1.2 eq.) was further added to the reaction solution. After the completion of the reaction was detected, the mixture was concentrated by rotary evaporation. 3 L of methanol and 3 L of concentrated aqueous ammonia were added into the rotary flask, and the mixture was allowed to react at room temperature for 4 h. After the completion of the reaction was detected, the mixture was concentrated by rotary evaporation. 20 L of ultrapure water was added, and the mixture was loaded into a reverse ion permeation device, washed, concentrated, and lyophilized to obtain intermediate D. The reaction route is shown in equation (6):

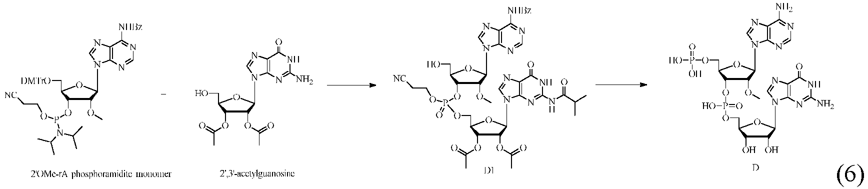

### Example 3: Synthesis method for vinylphosphonic acid-modified mRNA cap analog using intermediates A and E as starting materials

With reference to the synthesis method for the target product of Example 1, the vinylphosphonic acid-modified mRNA cap analog of Example 3 was obtained using intermediates A and E as starting materials. The reaction route is shown in equation (7):

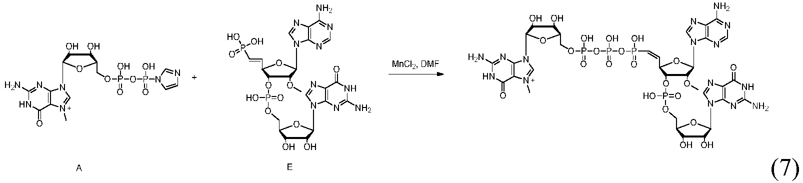

In the route, compound E was obtained by the following steps: 1) 5 g of intermediate D1 was dissolved in 50 mL of DMF, and DMSO (6.0 eq.) and EDCI (3.0 eq.) were added to the reaction solution before pyridine (1.0 eq.) and trifluoroacetic acid (1.0 eq.) were added dropwise. The mixture was allowed to react at room temperature for 5 h, and then the reaction solution was diluted with ethyl acetate. The organic phase was separately washed with a saturated aqueous sodium bicarbonate solution and water, then concentrated, and subjected to column chromatography to obtain intermediate E1.
2) Tetraethyl methylenediphosphonate (1.5 eq.) was added dropwise to a suspension of NaH (60%, 1.0 eq.) in THF (80 mL) under an ice bath, and then the mixture was stirred at 0 °C for 0.5 h. A solution of intermediate E1 (3 g) in THF (40 mL) was slowly added dropwise to the reaction solution. The mixture was stirred at room temperature overnight. A saturated aqueous ammonium chloride solution was added dropwise to the reaction solution under an ice bath to quench the reaction, and the mixture was extracted with ethyl acetate, then concentrated, and subjected to column chromatography to obtain intermediate E2.
3) 1 g of intermediate E2 was dissolved in 20 mL of acetonitrile, and triethylbromosilane (10.0 eq.) was added to the reaction solution. The mixture was heated to 75 °C, incubated overnight, and then concentrated. The crude product was subjected to reverse-phase preparative chromatography to obtain intermediate E3.
4) 0.3 g of intermediate E3 was dissolved in 6 mL of aqueous ammonia, and the solution was heated to 50 °C for reaction overnight. After the reaction was completed, the reaction solution was concentrated and purified by reverse-phase chromatography to obtain intermediate E. The reaction route is shown in equation (8):

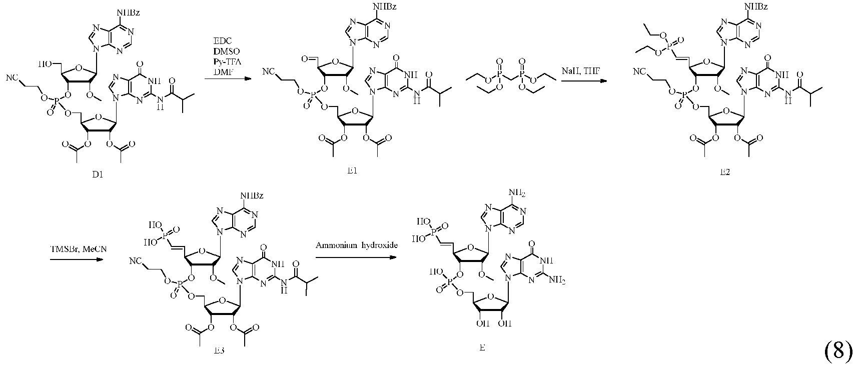

### Example 4: Synthesis method for vinylphosphonic acid-modified mRNA cap analog using intermediates B and C as starting materials

With reference to the synthesis method for the target product of Example 1, the vinylphosphonic acid-modified mRNA cap analog of Example 4 was obtained using intermediates B and C as starting materials. The reaction route is shown in equation (9):

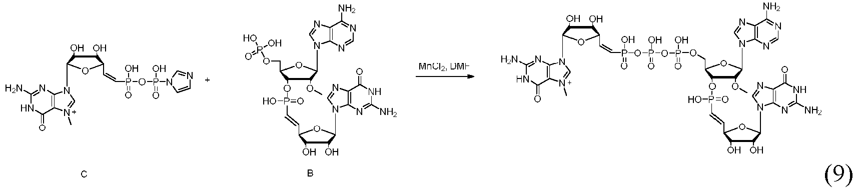

### Example 5: Synthesis method for vinylphosphonic acid-modified mRNA cap analog using intermediates C and E as starting materials

With reference to the synthesis method for the target product of Example 1, the vinylphosphonic acid-modified mRNA cap analog of Example 5 was obtained using intermediates C and E as starting materials. The reaction route is shown in equation (10):

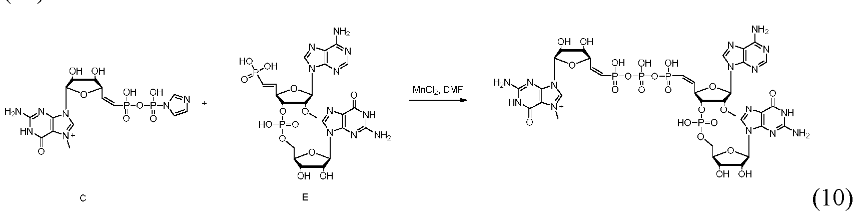

### Example 6: Synthesis method for vinylphosphonic acid-modified mRNA cap analog using intermediates A and F as starting materials

With reference to the synthesis method for the target product of Example 1, the vinylphosphonic acid-modified mRNA cap analog of Example 6 was obtained using intermediates A and F as starting materials. The reaction route is shown in equation (11):

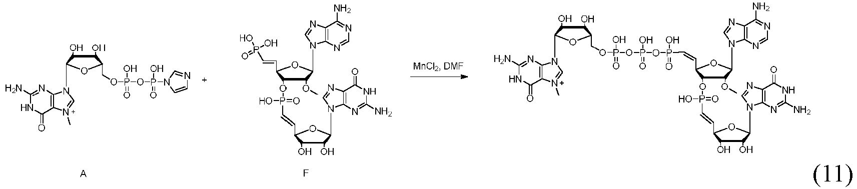

In the route, compound F was obtained by the following steps: 1) 5 g of intermediate B9 was dissolved in 50 mL of DMF, and DMSO (6.0 eq.) and EDCI (3.0 eq.) were added to the reaction solution before pyridine (1.0 eq.) and trifluoroacetic acid (1.0 eq.) were added dropwise. The mixture was allowed to react at room temperature for 5 h, and then the reaction solution was diluted with ethyl acetate. The organic phase was separately washed with a saturated aqueous sodium bicarbonate solution and water, then concentrated, and subjected to column chromatography to obtain intermediate E1.
2) Tetraethyl methylenediphosphonate (1.5 eq.) was added dropwise to a suspension of NaH (60%, 2.0 eq.) in THF (80 mL) under an ice bath, and then the mixture was stirred at 0 °C for 0.5 h. A solution of intermediate E1 (3 g) in THF (40 mL) was slowly added dropwise to the reaction solution. The mixture was stirred at room temperature overnight. The reaction was quenched with ice water under an ice bath, and the mixture was stirred at room temperature for 2 h and then concentrated. The crude product was purified by reverse-phase chromatography to obtain intermediate F2.
3) 1 g of intermediate F2 was dissolved in 20 mL of DMF, and triethylbromosilane (10.0 eq.) was added to the reaction solution. The mixture was heated to 75 °C, incubated overnight, and then concentrated. The crude product was subjected to reverse-phase preparative chromatography to obtain intermediate F3.
4) 0.3 g of intermediate F3 was dissolved in H₂O/TFA (1:1, 30 mL), and the solution was stirred at room temperature for 1 h. After the reaction was completed, the solution was purified by reverse-phase chromatography to obtain intermediate F. The reaction route is shown in equation (12):

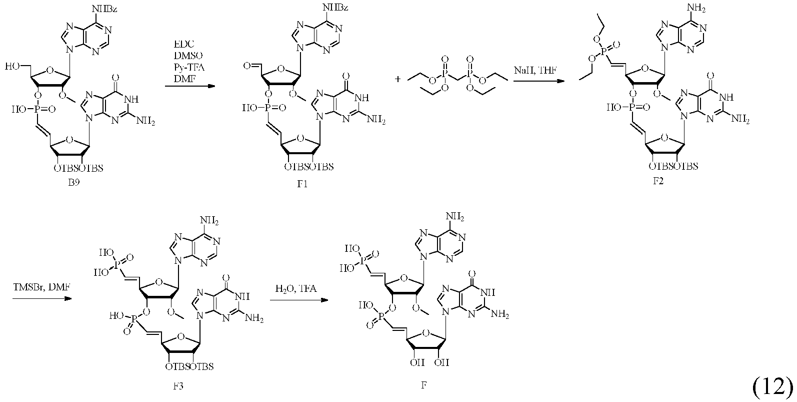

### Example 7: Synthesis method for vinylphosphonic acid-modified mRNA cap analog using intermediates C and F as starting materials

With reference to the synthesis method for the target product of Example 1, the vinylphosphonic acid-modified mRNA cap analog of Example 7 was obtained using intermediates C and F as starting materials. The reaction route is shown in equation (13):

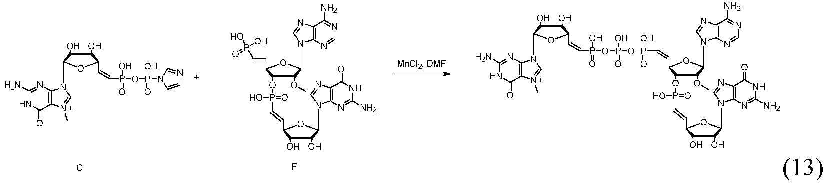

### Example 8: Synthesis method for vinylphosphonic acid-modified mRNA cap analog (compound 10) using intermediates D and G as starting materials

With reference to the synthesis method for the target product of Example 1, the vinylphosphonic acid-modified mRNA cap analog of Example 8 was obtained using intermediates D and G as starting materials. The reaction route is shown in equation (14):

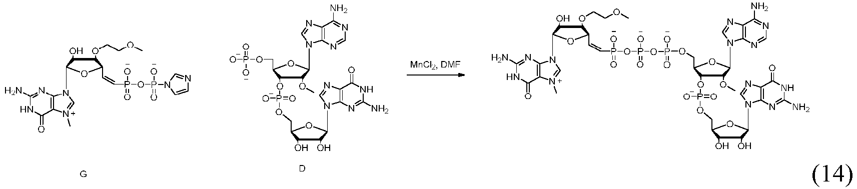

In the route, compound G was obtained by the following steps:
1) With reference to the synthesis methods for compounds B1, B2, B3, B4, B5 and B6, intermediates G2, G3, G4, G5, G6 and G7 were obtained, respectively.
2) 8 g of intermediate G7 was dissolved in 80 mL of acetonitrile, and triethylbromosilane (10.0 eq.) was added to the reaction solution. The mixture was stirred at room temperature overnight and then concentrated to obtain a crude product of intermediate G8, which was directly used in the next step without purification.
3) The crude product of intermediate G8 was dissolved in THF (10 V), and TBAF (3.0 eq.) was added at room temperature. The mixture was stirred at room temperature for 3 h and then concentrated under reduced pressure to obtain a crude product of intermediate G9, which was directly used in the next step without purification.
4) The crude product of intermediate G9 was dissolved in concentrated aqueous ammonia (10 V), and the solution was stirred at room temperature overnight. After the reaction was completed, the reaction solution was concentrated under reduced pressure and purified by reverse-phase chromatography to obtain intermediate G10.
5) With reference to the synthesis methods for compounds C2, C3, C4 and C, intermediates G11, G12, G13 and G were obtained, respectively. The reaction route is shown in equation (15):

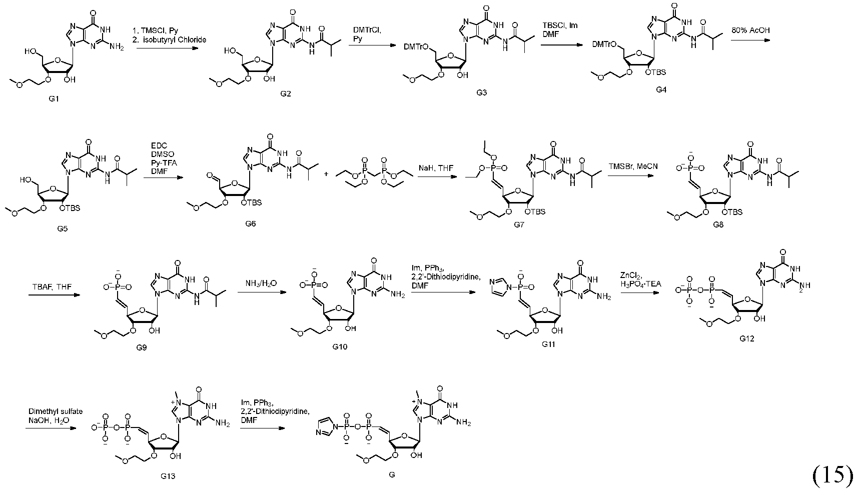

### Example 9: Synthesis method for vinylphosphonic acid-modified mRNA cap analog (compound 11) using intermediates D and H as starting materials

With reference to the synthesis method for the target product of Example 1, the vinylphosphonic acid-modified mRNA cap analog of Example 9 was obtained using intermediates D and H as starting materials. The reaction route is shown in equation (16):

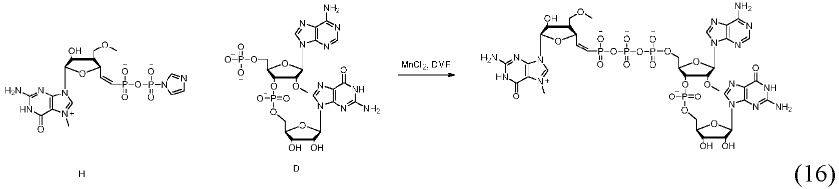

In the route, compound H was obtained by the following steps:
(1) Compound H1 (0.18 mol) and imidazole (0.36 mol) were dissolved in DMF (3.5 L). *tert*-Butyldiphenylchlorosilane (0.2 mol) was added to the reaction solution under an ice bath. The mixture was stirred at room temperature for 5 h. The reaction solution was then poured into ice water, and the mixture was extracted with ethyl acetate (500.0 mL × 2). The organic phase was washed with saturated sodium chloride, dried, and concentrated under reduced pressure to obtain intermediate H2, which was directly used in the next step without purification.
(2) Acetic anhydride (0.45 mol) and pyridine (0.8 mol) were slowly added to a suspension of CrO₃ (0.45 mol) in dichloromethane (900.0 mL) under an ice-salt bath. The mixture was stirred at room temperature for 15 min, and then intermediate H2 (0.15 mol) was added to the reaction solution. The reaction solution was stirred at room temperature for 1 h and then poured into iced ethyl acetate. The mixture was filtered under vacuum, and the filtrate was concentrated under reduced pressure and then purified by column chromatography to obtain intermediate H3.
(3) Methyltriphenylphosphonium bromide (0.15 mol) was added dropwise to a suspension of NaH (60%, 0.12 mol) in THF (500.0 mL) under an ice bath, and the mixture was stirred at 0 °C for 0.5 h. A solution of intermediate H3 (0.12 mol) in THF (300.0 mL) was slowly added dropwise to the reaction solution. The mixture was stirred at room temperature overnight. A saturated aqueous ammonium chloride solution was added dropwise to the reaction solution under an ice bath to quench the reaction, and the mixture was extracted with ethyl acetate, then concentrated, and purified by column chromatography to obtain intermediate H4.
(4) Intermediate H4 (0.1 mol) was dissolved in anhydrous THF (420.0 mL), and with the temperature controlled at -5 to 0 °C, the solution was added dropwise to a borane dimethyl sulfide solution (0.2 mol, 10 M) under nitrogen atmosphere. After the dropwise addition, the mixture was stirred for 6 h at a constant temperature. An aqueous sodium hydroxide solution (0.7 mol, 350.0 mL) and hydrogen peroxide (80.0 mL, 30% aqueous solution) were sequentially added dropwise to the reaction solution. The mixture was warmed to room temperature, stirred for 2 h, and extracted with ethyl acetate (200.0 mL × 2). The organic phase was washed sequentially with water and saturated brine, dried over anhydrous sodium sulfate, concentrated under reduced pressure, and purified by column chromatography to obtain intermediate H5.
(5) A solution of intermediate H5 (85.0 mmol) in THF (200.0 mL) was added dropwise to a suspension of NaH (60%, 95.0 mmol) in THF (170.0 mL) under an ice bath, and the mixture was stirred at 0 °C for 0.5 h. Methyl iodide (100.0 mmol) was added dropwise to the reaction solution. The mixture was stirred at room temperature for 3 h. A saturated aqueous ammonium chloride solution was added dropwise to the reaction solution under an ice bath to quench the reaction, and the mixture was extracted with ethyl acetate, then concentrated, and purified by column chromatography to obtain intermediate H6.
(6) Intermediate H6 (70.0 mmol) was dissolved in anhydrous THF (300.0 mL), and a solution of TBAF in THF (1 M, 0.3 mol) was added. The mixture was then stirred at room temperature for 6 h and washed with water. The aqueous phase was further extracted once with ethyl acetate. The organic phases were combined, dried, concentrated, and purified by column chromatography to obtain intermediate H7.
(7) Intermediate H7 (60.0 mmol) was dissolved in DMF (130.0 mL), and DMSO (0.36 mol) and EDCI (0.18 mol) were added to the reaction solution before pyridine (60.0 mmol) and trifluoroacetic acid (60.0 mmol) were added dropwise. The mixture was allowed to react at room temperature for 5 h, and then the reaction solution was diluted with ethyl acetate. The organic phase was separately washed with a saturated aqueous sodium bicarbonate solution and water, then concentrated, and subjected to column chromatography to obtain intermediate H8.
(8) Tetraethyl methylenediphosphonate (80.0 mmol) was added dropwise to a suspension of NaH (60%, 53.0 mmol) in THF (50.0 mL) under an ice bath, and then the mixture was stirred at 0 °C for 0.5 h. A solution of intermediate H8 (53.0 mmol) in THF (70.0 mL) was slowly added dropwise to the reaction solution. The mixture was stirred at room temperature overnight. A saturated aqueous ammonium chloride solution was added dropwise to the reaction solution under an ice bath to quench the reaction, and the mixture was extracted with ethyl acetate, then concentrated, and subjected to column chromatography to obtain intermediate H9.
(9) Intermediate H9 (44.0 mmol) was dissolved in acetic acid (80.0 mL), and acetic anhydride (132.0 mmol) was added at room temperature. The mixture was cooled to below 10 °C, and concentrated sulfuric acid (6.6 mmol) was added dropwise to the reaction solution. The mixture was stirred at room temperature overnight, diluted with DCM (150.0 mL), then washed sequentially with water, a saturated aqueous sodium bicarbonate solution and saturated brine, and dried over anhydrous sodium sulfate. The organic phase was concentrated under reduced pressure to obtain intermediate H10, which was directly used in the next step without purification.
(10) Compound H10 (40.0 mmol) and compound H11 (40.0 mmol) were weighed and suspended in DCE (300.0 mL), and BSA (0.1 mol) was added to the reaction solution. The reaction mixture was heated to 70 °C, stirred for 1 h, and then cooled to 0 °C. TMSOTf (0.12 mol) was added dropwise to the reaction solution. After the dropwise addition, the mixture was heated to 70 °C, stirred for 3 h, then cooled to room temperature, and washed with a saturated aqueous sodium bicarbonate solution. The organic phase was concentrated under reduced pressure and then purified by column chromatography to obtain intermediate H12.
(11) With reference to the synthesis method for intermediate G8, intermediate H13 was obtained.
(12) With reference to the synthesis method for intermediate G10, intermediate H14 was obtained.
(13) With reference to the synthesis methods for compounds G11, G12, G13 and G, intermediates H15, H16, H17 and H were obtained, respectively. The reaction route is shown in equation (17):

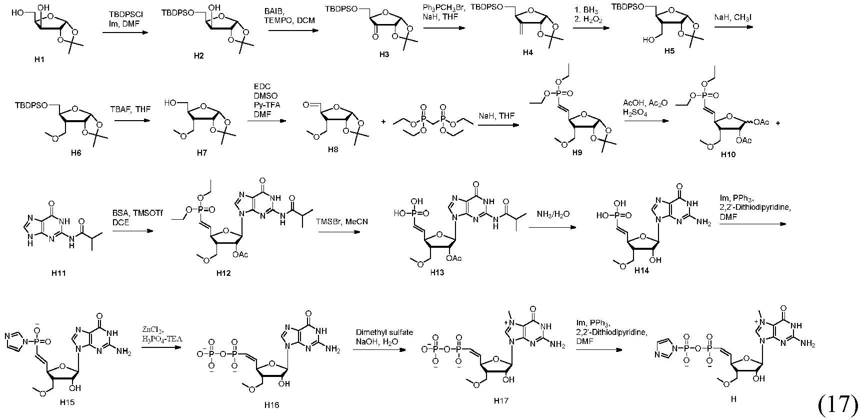

### Example 10: Synthesis method for vinylphosphonic acid-modified mRNA cap analog (compound 12) using intermediates D and I as starting materials

With reference to the synthesis method for the target product of Example 1, the vinylphosphonic acid-modified mRNA cap analog of Example 10 was obtained using intermediates D and I as starting materials. The reaction route is shown in equation (18):

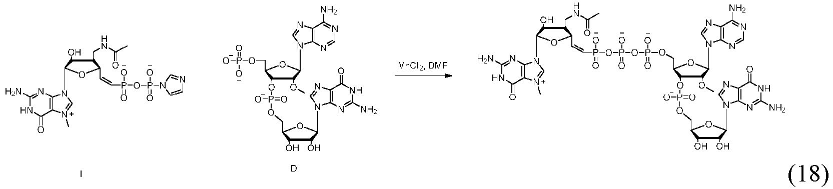

In the route, compound I was obtained by the following steps:
1) Intermediate H5 (0.33 mol), triphenylphosphine (0.66 mol), and DIAD (0.66 mol) were dissolved in THF (1.5 L). DPPA (0.66 mol) was added dropwise to the reaction solution. The mixture was stirred at room temperature overnight, concentrated under reduced pressure to remove the solvent, and purified by column chromatography to obtain intermediate I1.
2) With reference to the synthesis method for intermediate H7, intermediate I2 was obtained.
3) With reference to the synthesis method for intermediate H8, intermediate I3 was obtained.
4) With reference to the synthesis method for intermediate H9, intermediate I4 was obtained.
5) With reference to the synthesis method for intermediate H10, intermediate I5 was obtained.
6) With reference to the synthesis method for intermediate H12, intermediate I6 was obtained.
7) With reference to the synthesis method for intermediate H14, intermediate I7 was obtained.
8) Intermediate I7 (55.0 mmol) was dissolved in THF (500.0 mL). Water (50.0 mL) and triphenylphosphine (82.5 mmol) were then added, and the mixture was heated to 50 °C, stirred overnight, and concentrated to remove the solvent. The crude product was purified by column chromatography to obtain intermediate I8.
9) Intermediate I8 (50.0 mmol) was dissolved in DCM (100.0 mL) and pyridine (100.0 mL). The solution was cooled to -10 °C, and acetyl chloride (55.0 mmol) was added dropwise. The mixture was then stirred for 3 h, and methanol (3.0 mL) was added to quench the reaction. The mixture was concentrated under reduced pressure and then purified by column chromatography to obtain intermediate I9.
10) With reference to the synthesis method for intermediate H13, intermediate I10 was obtained.
11) With reference to the synthesis method for intermediate H15, intermediate I11 was obtained.
12) With reference to the synthesis method for intermediate H16, intermediate I12 was obtained.
13) With reference to the synthesis method for intermediate H17, intermediate I13 was obtained.
14) With reference to the synthesis method for intermediate H, intermediate I was obtained. The reaction route is shown in equation (19):

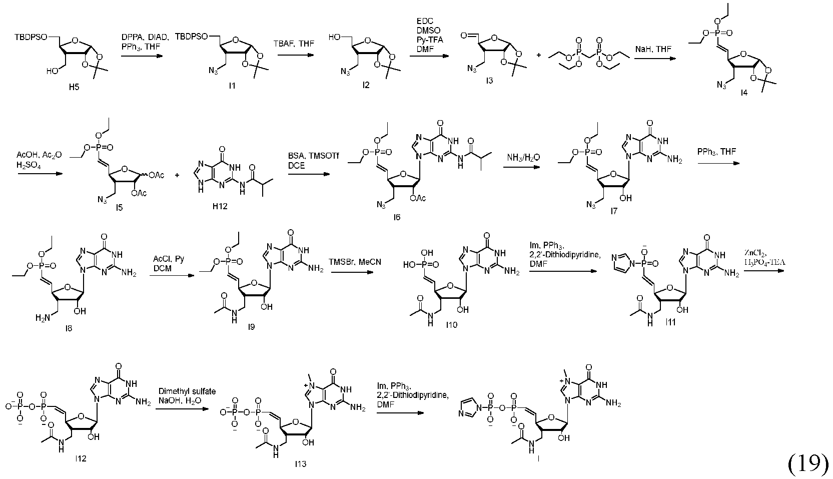

### Example 11: Synthesis method for vinylphosphonic acid-modified mRNA cap analog (compound 13) using intermediates D and J as starting materials

With reference to the synthesis method for the target product of Example 1, the vinylphosphonic acid-modified mRNA cap analog of Example 11 was obtained using intermediates D and J as starting materials. The reaction route is shown in equation (20):

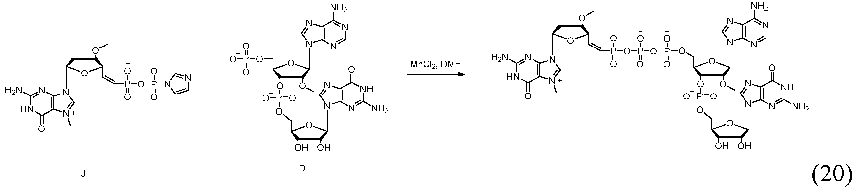

In the route, compound J was obtained by the following steps:
1) Intermediate J1 (80.0 mmol) was dissolved in pyridine (520.0 mL), and phenyl chlorothionocarbonate (0.24 mol) was added to the reaction solution at room temperature. The mixture was then stirred at room temperature overnight, concentrated under reduced pressure to remove the solvent, and then purified by column chromatography to obtain intermediate J2.
2) Intermediate J2 (75.0 mmol) and AIBN (15.0 mol) were dissolved in toluene (590.0 mL). The reaction solution was heated to reflux, and tributyltin hydride (0.23 mol) was added. The mixture was stirred for another 2 h, concentrated under reduced pressure to remove the solvent, and then purified by column chromatography to obtain intermediate J3.
3) Intermediate J3 (42.0 mmol) was dissolved in DCM (270.0 mL). Trichloroacetic acid (0.13 mol) was added to the reaction solution at room temperature, and the mixture was stirred at room temperature for 2 h and separately washed with water and saturated sodium bicarbonate. The organic phase was concentrated under reduced pressure and then purified by column chromatography to obtain intermediate J4.
4) With reference to the synthesis method for intermediate G6, intermediate J5 was obtained.
5) With reference to the synthesis method for intermediate G7, intermediate J6 was obtained.
6) With reference to the synthesis method for intermediate G8, intermediate J7 was obtained.
7) With reference to the synthesis method for intermediate I7, intermediate J8 was obtained.
8) With reference to the synthesis method for intermediate I11, intermediate J9 was obtained.
9) With reference to the synthesis method for intermediate I12, intermediate J10 was obtained.
10) With reference to the synthesis method for intermediate I13, intermediate J11 was obtained.
11) With reference to the synthesis method for intermediate I, intermediate J was obtained. The reaction route is shown in equation (21):

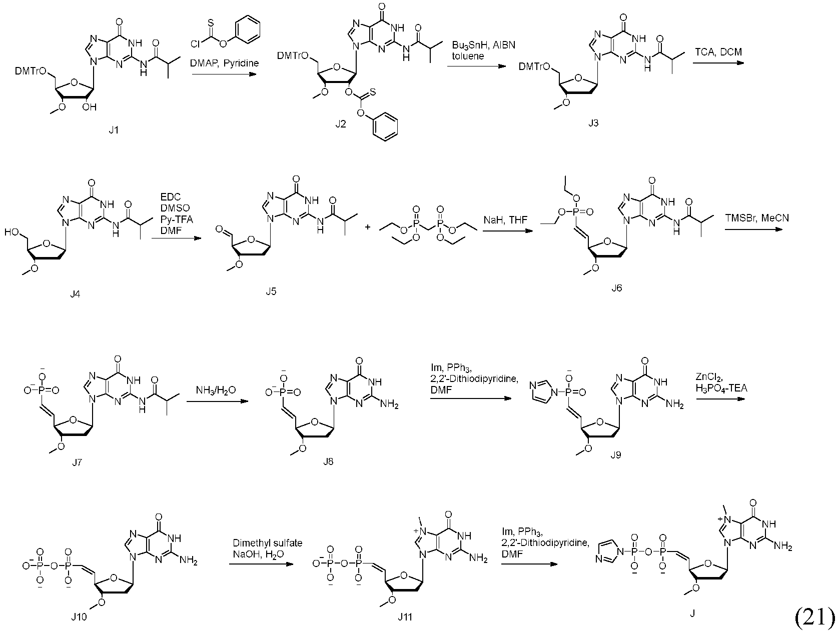

### Example 12: Synthesis method for vinylphosphonic acid-modified mRNA cap analog (compound 15) using intermediates D and K as starting materials

With reference to the synthesis method for the target product of Example 1, the vinylphosphonic acid-modified mRNA cap analog of Example 12 was obtained using intermediates D and K as starting materials. The reaction route is shown in equation (22):

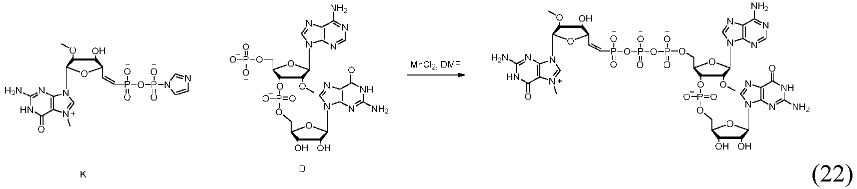

In the route, compound K was obtained by the following steps: With reference to the synthesis methods for intermediates G4 to G, intermediates K2 to K were sequentially obtained. The reaction route is shown in equation (23):

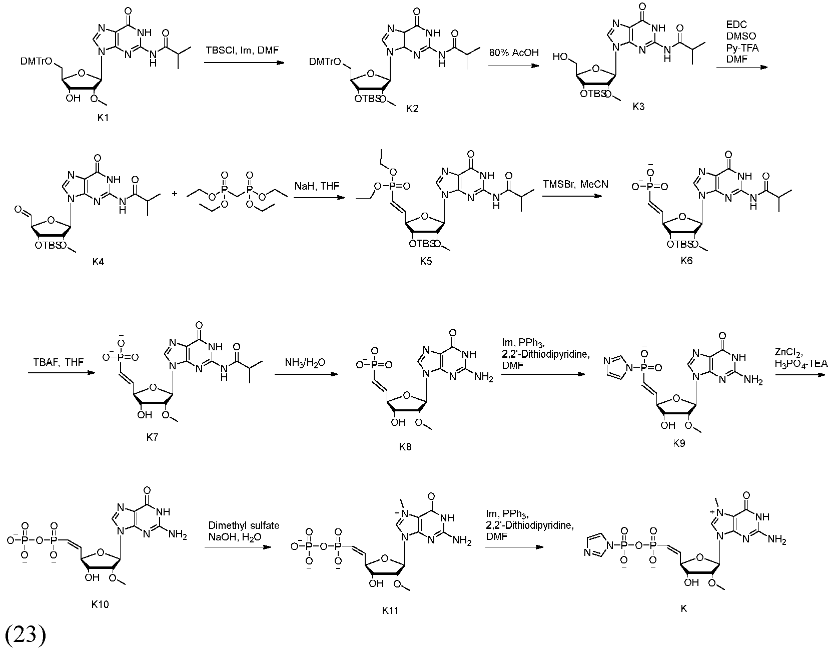

### Example 13: Synthesis method for vinylphosphonic acid-modified mRNA cap analog (compound 17) using intermediates I and L as starting materials

With reference to the synthesis method for the target product of Example 1, the vinylphosphonic acid-modified mRNA cap analog of Example 13 was obtained using intermediates I and L as starting materials. The reaction route is shown in equation (24):

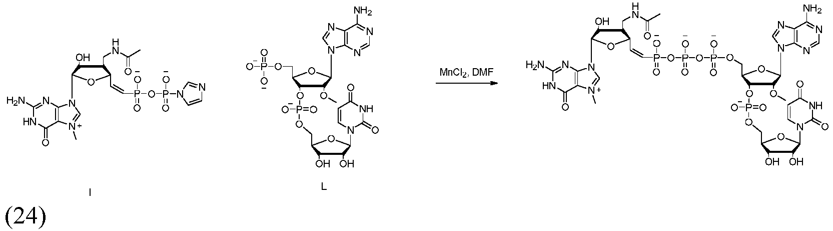

In the route, compound L was obtained by the following steps: With reference to the synthesis method for intermediate D of Example 2, intermediate L was obtained. The reaction route is shown in equation (25) below:

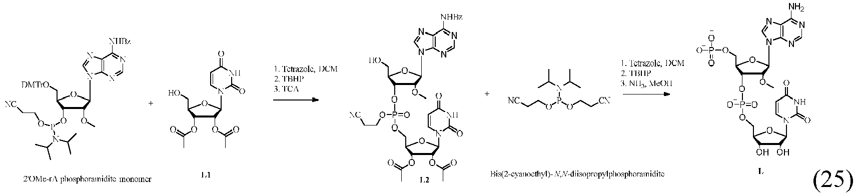

### Example 14: Synthesis method for vinylphosphonic acid-modified mRNA cap analog (compound 25) using intermediates E and M as starting materials

With reference to the synthesis method for the target product of Example 1, the vinylphosphonic acid-modified mRNA cap analog of Example 14 was obtained using intermediates E and M as starting materials. The reaction route is shown in equation (26):

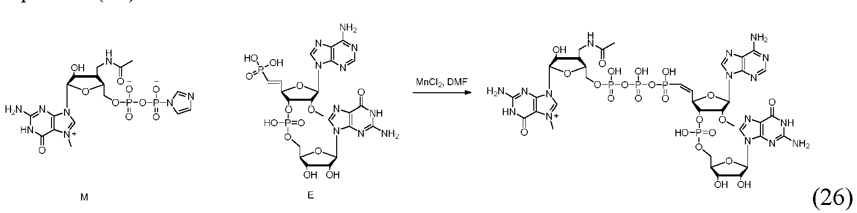

In the route, compound M was obtained by the following steps:
1) With reference to the synthesis method for intermediate I5, intermediate M1 was obtained.
2) With reference to the synthesis method for intermediate I6, intermediate M2 was obtained.
3) With reference to the synthesis method for intermediate I7, intermediate M3 was obtained.
4) With reference to the synthesis method for intermediate I8, intermediate M4 was obtained.
5) With reference to the synthesis method for intermediate I9, intermediate M5 was obtained.
6) With reference to the synthesis method for intermediate H7, intermediate M6 was obtained.
7) With reference to the synthesis method for intermediate A1, intermediate M7 was obtained.
8) With reference to the synthesis method for intermediate K9, intermediate M8 was obtained.
9) With reference to the synthesis method for intermediate K10, intermediate M9 was obtained.
10) With reference to the synthesis method for intermediate K11, intermediate M10 was obtained.
11) With reference to the synthesis method for intermediate K, intermediate M was obtained. The reaction route is shown in equation (27):

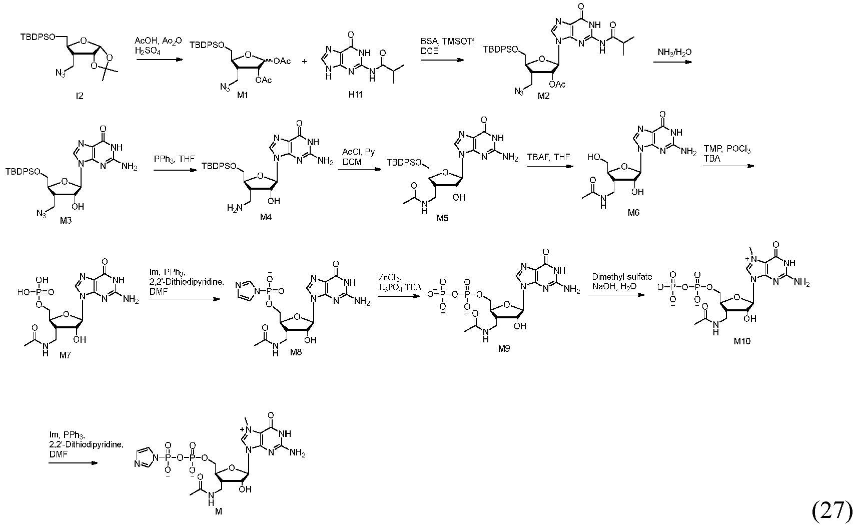

### Example 15: Synthesis method for vinylphosphonic acid-modified mRNA cap analog (compound 31) using intermediates D and N as starting materials

With reference to the synthesis method for the target product of Example 1, the vinylphosphonic acid-modified mRNA cap analog of Example 15 was obtained using intermediates D and N as starting materials. The reaction route is shown in equation (28):

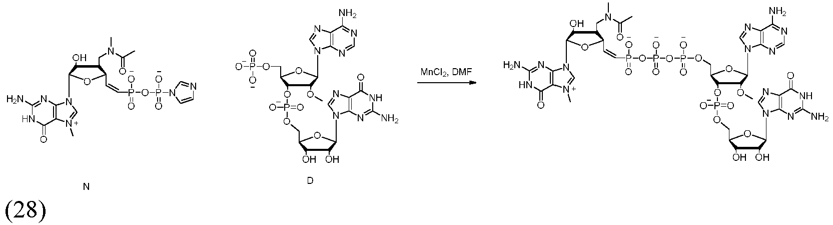

In the route, compound N was obtained by the following steps:
1) With reference to the synthesis method for intermediate M4, intermediate N1 was obtained.
2) With reference to the synthesis method for intermediate M5, intermediate N2 was obtained.
3) With reference to the synthesis method for intermediate M3, intermediate N3 was obtained.
4) With reference to the synthesis method for intermediate M5, intermediate N4 was obtained.
5) With reference to the synthesis method for intermediate K6, intermediate N5 was obtained.
6) With reference to the synthesis method for intermediate M8, intermediate N6 was obtained.
7) With reference to the synthesis method for intermediate M9, intermediate N7 was obtained.
8) With reference to the synthesis method for intermediate M10, intermediate N8 was obtained.
9) With reference to the synthesis method for intermediate M, intermediate N was obtained.

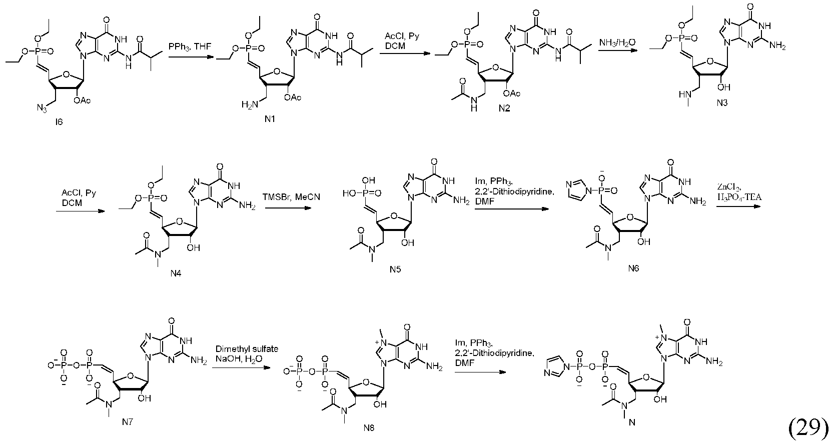

### Example 16: Synthesis method for vinylphosphonic acid-modified mRNA cap analog (compound 34) using intermediates D and O as starting materials

With reference to the synthesis method for the target product of Example 1, the vinylphosphonic acid-modified mRNA cap analog of Example 16 was obtained using intermediates D and O as starting materials. The reaction route is shown in equation (30):

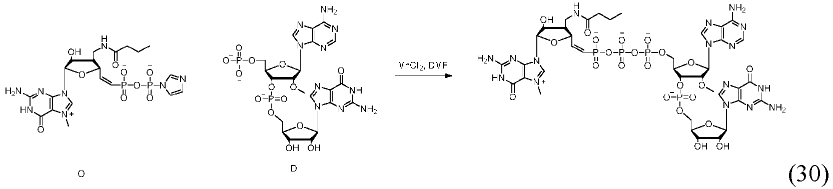

In the route, compound O was obtained by the following steps: With reference to the synthesis methods for intermediates I9, I10, I11, I12, I13 and I, intermediates O1, O2, O3, O4, O5 and O were obtained, respectively.

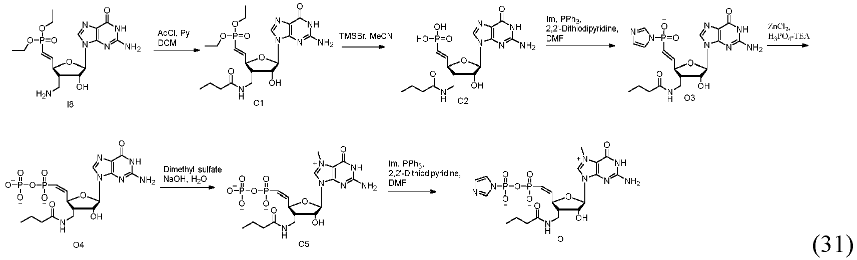

### Example 17: Synthesis method for vinylphosphonic acid-modified mRNA cap analog (compound 16) using intermediates D and P as starting materials

With reference to the synthesis method for the target product of Example 1, the vinylphosphonic acid-modified mRNA cap analog of Example 17 was obtained using intermediates D and P as starting materials. The reaction route is shown in equation (32):

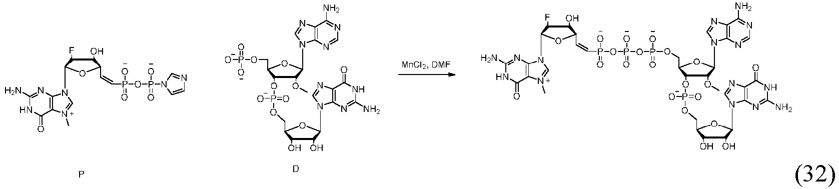

In the route, compound P was obtained by the following steps: With reference to the synthesis methods for intermediates G4 to G, intermediates P2 to P were sequentially obtained. The reaction route is shown in equation (33):

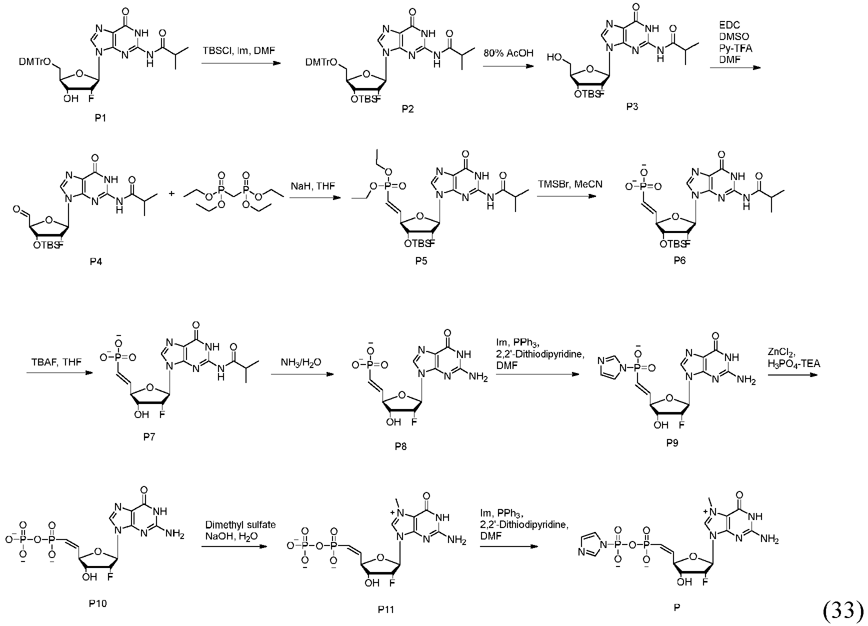

### Comparative Example 1: ^{m7}GpppA_{2'OMe}pG

With reference to the synthesis methods in the above examples, ^{m7}GpppA_{2'OMe}pG was synthesized (starting materials used referred to the preparation methods in the examples). The reaction route is shown in equation (34):

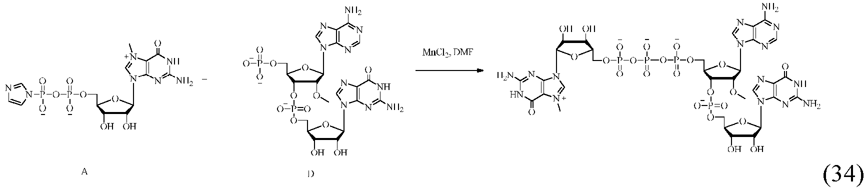

The structures of the cap analogs obtained in the examples and the comparative example are shown in Table 2 below.

**Table 2**

| No. | Cap analog |
|---|---|
| Example 1 | 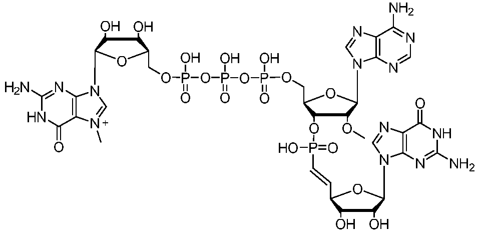 |
| Example 2 | 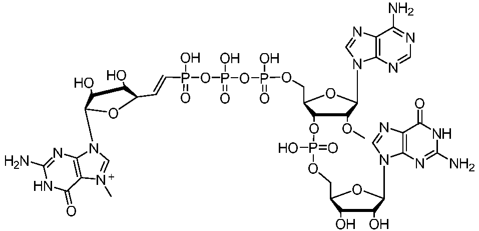 |
| Example 3 | 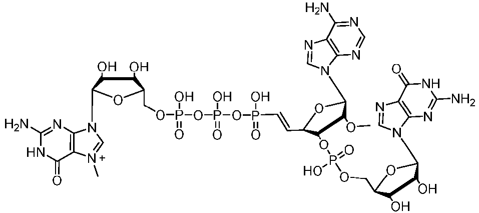 |
| Example 4 | 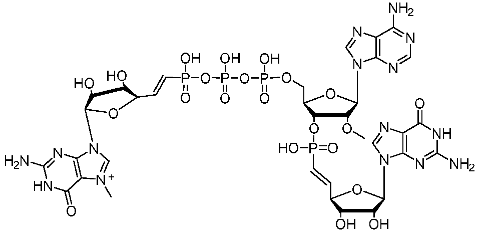 |
| Example 5 | 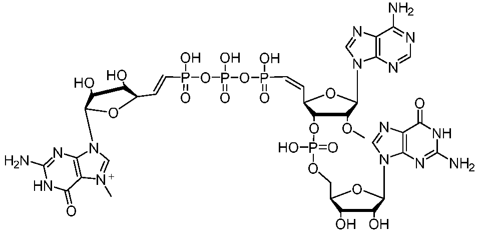 |
| Example 6 | 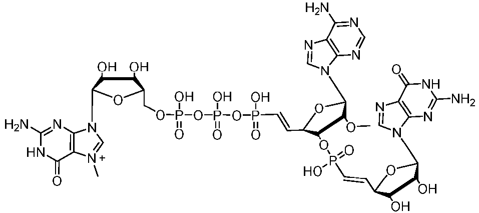 |
| Example 7 | 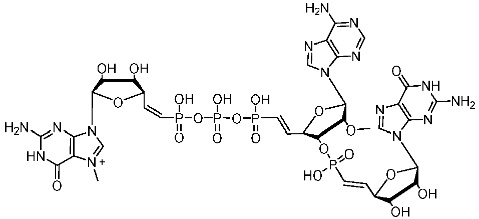 |
| Compound 8 | 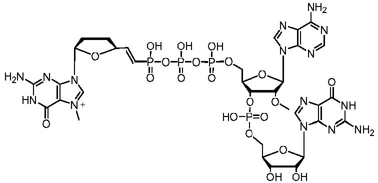 |
| Compound 10 | 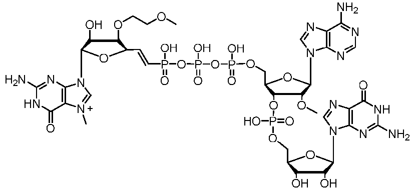 |
| Compound 11 | 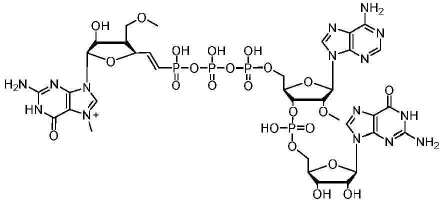 |
| Compound 12 | 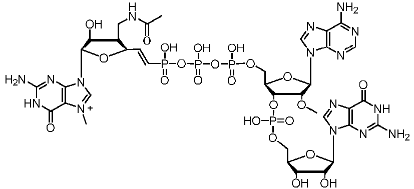 |
| Compound 13 | 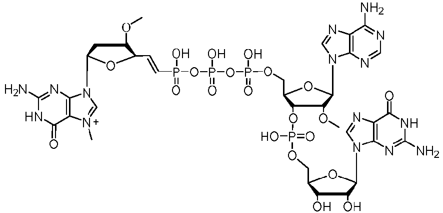 |
| Compound 14 | 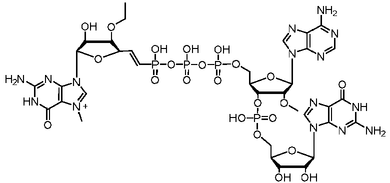 |
| Compound 16 | 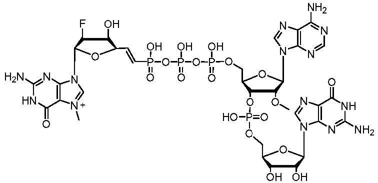 |
| Compound 17 | 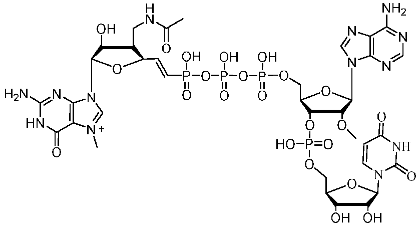 |
| Compound 18 | 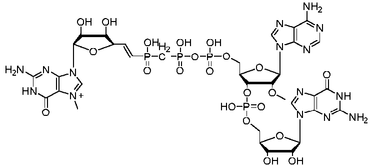 |
| Compound 19 | 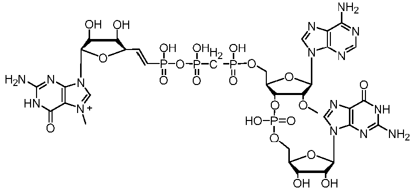 |
| Compound 20 | 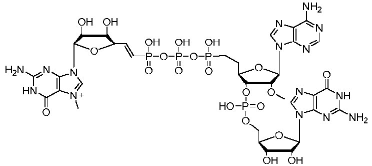 |
| Compound 21 | 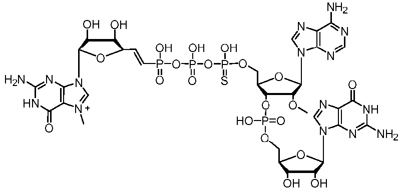 |
| Compound 22 | 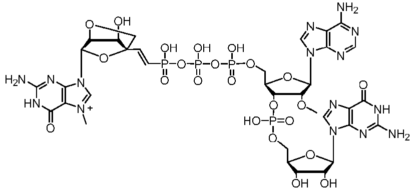 |
| Compound 23 | 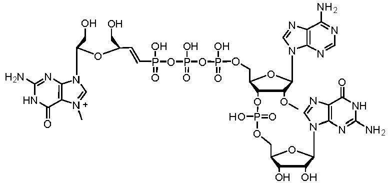 |
| Compound 24 | 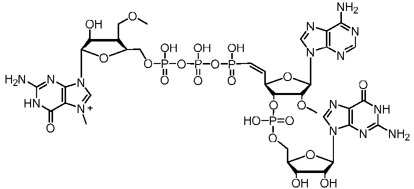 |
| Compound 25 | 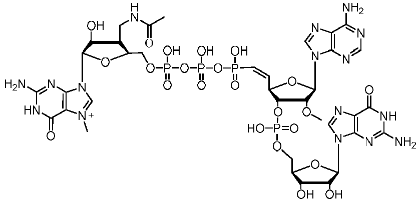 |
| Compound 26 | 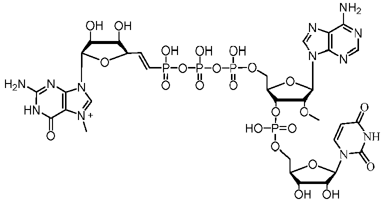 |
| Compound 27 | 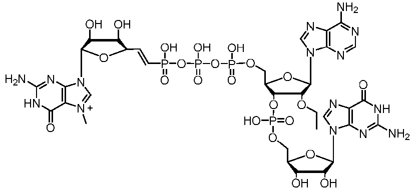 |
| Compound 28 | 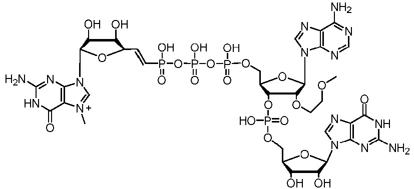 |
| Compound 30 | 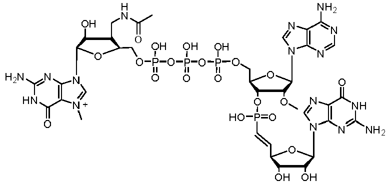 |
| Compound 31 | 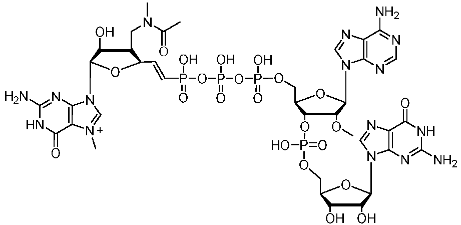 |
| Compound 32 | 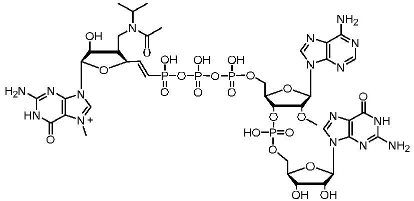 |
| Compound 33 | 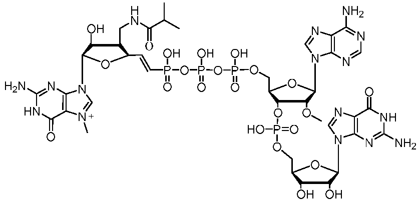 |
| Compound 34 | 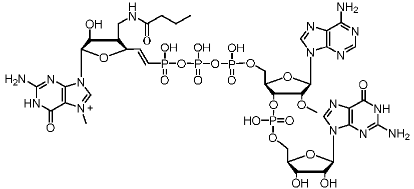 |
| Compound 35 | 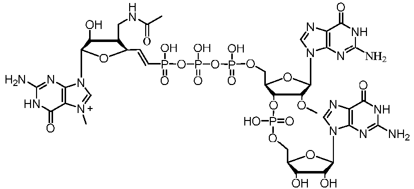 |
| Comparative Example 1 | 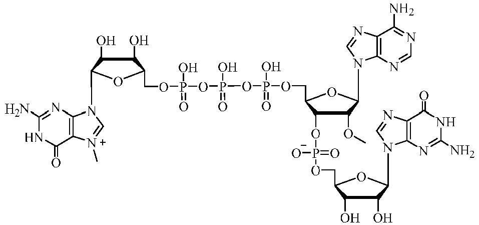 |

### Test Example 1: Determination of mRNA in-vitro transcription yield and capping efficiency

*In-vitro* synthesis of mRNA cap analogs modified with vinylphosphonic acid: Firstly, a plasmid was linearized with NotI and digested at 4 °C overnight. A DNA template was extracted. mRNAs were synthesized by *in-vitro* transcription using the vinylphosphonic acid-modified mRNA cap analogs of Examples 1-7 and compounds 10-13, 16, 17, 25, 31 and 34, and the cap analog of Comparative Example 1 as cap structures, respectively.

The reaction system is shown in Table 3:

**Table 3**

| System | Amount |
|---|---|
| T7 RNA polymerase | 50 U |
| 10X buffer | 2 µl |
| 100mM ATP | 1 µl |
| 100mM GTP | 1 µl |
| 100mM CTP | 1 µl |
| 100mM N1-Me-pUTP | 1 µl |
| 100 mM cap analog | 1 µl |
| Inorganic pyrophosphatase | 0.05 U |
| Nuclease inhibitor | 20 U |
| Sterile enzyme-free water | Making up to 20 µL |
| Template | 1 µg |

| | |
|---|---|
| Note: During the experiment, the volume of the materials required for the system was calculated firstly, and then sample loading was performed. Firstly, sterile enzyme-free water was added into the system, and then 10× buffer, NTPs, and the cap analog were sequentially added. The mixture was uniformly mixed and then gently centrifuged, followed by addition of the nuclease inhibitor, the inorganic pyrophosphatase, the T7 RNA polymerase, and the linearized DNA template. The mixture was well mixed, then gently centrifuged, and incubated at 37 °C. After 2 h, DNase I (1 U) was added, and the mixture was incubated at 37 °C for another 30 min to remove the DNA template. RNA purification was then performed (usually using the magnetic bead purification method). The purified mRNA was dissolved in sterile enzyme-free water, and then quantitative detection was performed by using Nanodrop One. | |

Liquid chromatography-mass spectrometry (LC-MS) was used to detect the IVT capping rates of mRNAs with different starting cap analogs. Firstly, a labeled (usually biotin-labeled) DNA probe matched with the starting base of mRNA as the transcript product was required to be designed, and streptavidin-labeled magnetic beads were washed, and then incubated with the synthesized DNA probe, mRNA and 10× RNase H reaction buffer with slowly mixing at room temperature for 30 min. 20 µL of RNase H (5 U/µL) was then added, and the mixture was incubated at 37 °C for 3 h, with uniform mixing every half hour. After the incubation was completed, the magnetic beads were cleaned, and then 100 µL of 75% methanol heated to 80 °C was added to the cleaned magnetic beads. The mixture was heated to 80 °C on a heating plate, held for 3 min, and then placed on a magnetic rack. The supernatant was pipetted and dried at room temperature for 45 min to 10 µL by using an evaporation centrifuge. The sample was then resuspended in 50 µL of 100 µM EDTA/1% MeOH for LC-MS analysis to determine the capping of RNA in the transcription reaction. Due to the significant difference in molecular weight between capped and uncapped bases, the capping rates of mRNA transcription initiated with different cap analogs can be determined by using the difference in molecular weight. The specific results are shown in Table 4.

**Table 4**

| No. | Yield (µg) | Capping rate (%) |
|---|---|---|
| Example 1 | 117 | 96.4 |
| Example 2 | 125 | 96.3 |
| Example 3 | 122 | 95.3 |
| Example 4 | 106 | 94.5 |
| Example 5 | 102 | 90.2 |
| Example 6 | 96 | 87.3 |
| Example 7 | 85 | 82.6 |
| Compound 10 | 124 | 96.4 |
| Compound 11 | 116 | 95.7 |
| Compound 12 | 123 | 96.6 |
| Compound 13 | 112 | 95.4 |
| Compound 16 | 114 | 95.8 |
| Compound 17 | 122 | 96.2 |
| Compound 25 | 120 | 96.1 |
| Compound 31 | 115 | 95.7 |
| Compound 34 | 117 | 95.9 |
| Comparative Example 1 | 120 | 95.6 |

As can be seen from the experimental results, Examples 1-7 and compounds 10-13, 16, 17, 25, 31 and 34 were all able to yield the corresponding target mRNAs by IVT. Among them, the yields of Examples 1, 2 and 3 and compounds 10-13, 16, 17, 25, 31 and 34 were comparable to that of Comparative Example 1, while the capping efficiencies of Examples 1, 2 and 3 and compounds 10-13, 16, 17, 25, 31 and 34 were better than that of Comparative Example 1 or comparable to that of Comparative Example 1. The *in-vitro* transcription yields and capping efficiencies of Examples 4, 5, 6 and 7 were significantly lower than those of Comparative Example 1. This may be related to the structures of the cap analogs, as the rigid structures of Examples 4, 5, 6 and 7 affected the binding of the cap analogs to the template transcription start site, thereby ultimately affecting the *in-vitro* transcription yields and capping efficiencies.

### Test Example 2: Assay of binding ability to eukaryotic initiation factor (elF4E)

Test method: Firstly, experimental samples, including the cap analog samples of Examples 1-7, compounds 10-13, 16, 17, 25, 31 and 34, and Comparative Example 1, were diluted with a running buffer (50 mM phosphate, 100 mM sodium chloride, and 0.01% vol/vol Tween 20, pH 6.0 or 7.4). The sample injection time was 2 min, the sample injection flow rate was 20 µL/min, and the dissociation time was 2.5 min. The residual samples were eluted with a regeneration buffer (10 mM HEPES, 150 mM NaCl, and 0.01% vol/vol Tween 20, pH 7.4) for 30 s at a flow rate of 30 µL/min. Finally, through analysis by Biacore T100 evaluation software (version 2.0.2), sensorgrams were generated and the binding constants were calculated.

**Table 5**

| No. | Binding constant (L/mol) |
|---|---|
| Example 1 | 3.19 x 10⁶ |
| Example 2 | 4.63 x 10⁶ |
| Example 3 | 4.16 x 10⁶ |
| Example 4 | 2.65 x 10⁶ |
| Example 5 | 2.53 x 10⁶ |
| Example 6 | 2.76 x 10⁶ |
| Example 7 | 0.86 x 10⁶ |
| Compound 10 | 4.58×10⁶ |
| Compound 11 | 4.07×10⁶ |
| Compound 12 | 4.68×10⁶ |
| Compound 13 | 3.77×10⁶ |
| Compound 16 | 3.72×10⁶ |
| Compound 17 | 4.51×10⁶ |
| Compound 25 | 4.47×10⁶ |
| Compound 31 | 4.19×10⁶ |
| Compound 34 | 4.23×10⁶ |
| Comparative Example 1 | 2.18 x 10⁶ |

As can be seen from the experimental data in Table 5 above, the binding constants to elF4E protein of Examples 1, 2, 3, 4, 5 and 6 and compounds 10-13, 16, 17, 25, 31 and 34 were better than that of Comparative Example 1, wherein the binding constants to elF4E protein of Examples 1, 2 and 3 and compounds 10-13, 16, 17, 25, 31 and 34 were significantly better than that of Comparative Example 1, indicating that Examples 1, 2 and 3 and compounds 10-13, 16, 17, 25, 31 and 34 had the strongest binding ability to elF4E protein. At the same time, we also found that the binding constant to elF4E protein of Example 7 was the lowest as compared to Comparative Example 1, indicating that Example 7 had the weakest binding ability to elF4E protein. Therefore, when a cap analog structure contains a vinyl structure, it may improve the binding ability of the cap analog to elF4E protein to a certain extent. However, the rigid structure of Example 7 containing three vinyl structures affected the binding of the cap analog to elF4E protein, thereby ultimately affecting the *in-vitro* transcription yield and capping efficiency.

### Test Example 3: mRNA translation efficiency

Test method: *In-vitro* transcription was initiated with the cap analogs of Examples 1-7, compounds 10-13, 16, 17, 25, 31 and 34, and Comparative Example 1 by using an eGFP coding sequence as a DNA template. The different mRNA products obtained were then transfected into 293T cells. The 293T cells were plated (onto a 24-well plate) at (0.5-1) × 10⁵ cells/well. During the transfection, in general, the cell density was preferably 60%-80%; 2 µg of mRNA was transfected into each well, and the Lipofectamine MessengerMAX Transfection Reagent (Invitrogen) was selected as the transfection reagent and used according to its usage instructions. The transfected cells were placed in an incubator at 37 °C with CO₂. After transfection for 4-6 h, the medium was replaced with a fresh complete medium. After incubation in the incubator at 37 °C with CO₂ for 24 h, the fluorescence intensity of GFPs in the cells was observed through a fluorescence microscope, and the fluorescence intensity ratios of Examples 1-7 and compounds 10-13, 16, 17, 25, 31 and 34 relative to Comparative Example 1 were calculated based on the fluorescence intensities.

**Table 6**

| No. | Cell fluorescence relative intensity (relative to Comparative Example 1) |
|---|---|
| Example 1 | 3.16 |
| Example 2 | 2.44 |
| Example 3 | 2.31 |
| Example 4 | 1.41 |
| Example 5 | 1.36 |
| Example 6 | 1.12 |
| Example 7 | 0.54 |
| Compound 10 | 3.12 |
| Compound 11 | 2.94 |
| Compound 12 | 3.06 |
| Compound 13 | 2.53 |
| Compound 16 | 2.49 |
| Compound 17 | 2.92 |
| Compound 25 | 2.85 |
| Compound 31 | 2.82 |
| Compound 34 | 2.84 |
| Comparative Example 1 | 1 |

As can be seen from the experimental data in Table 6 above, the intracellular translation effects of mRNAs transcribed by the cap analogs of Examples 1, 2, 3, 4, 5 and 6 and compounds 10-13, 16, 17, 25, 31 and 34 were better than that of Comparative Example 1, wherein the translation effects of Examples 1, 2 and 3 and compounds 10-13, 16, 17, 25, 31 and 34 were significantly better than that of Comparative Example 1, indicating that the mRNAs transcribed by the cap analogs of Examples 1, 2 and 3 and compounds 10-13, 16, 17, 25, 31 and 34 had the strongest intracellular translation effects. At the same time, we also found that the translation effect of Example 7 was the lowest as compared to Comparative Example 1. Therefore, when a cap analog structure contains a vinyl structure, it may have a promoting effect on improving the intracellular translation effect of mRNA with the cap analog. However, the rigid structure of Example 7 containing three vinyl structures affected the binding of the cap analog to elF4E protein, thereby ultimately affecting the intracellular translation effect of mRNA.

Although the embodiments of the present invention have been shown and described, it will be understood by those of ordinary skill in the art that various changes, modifications, substitutions and alterations may be made to these embodiments without departing from the principle and spirit of the present invention, and the scope of the present invention is defined in the appended claims and equivalents thereof.

## Claims

1. A vinylphosphonic acid-modified mRNA cap analog, comprising the following structure, or a pharmaceutically acceptable salt or stereoisomer thereof: 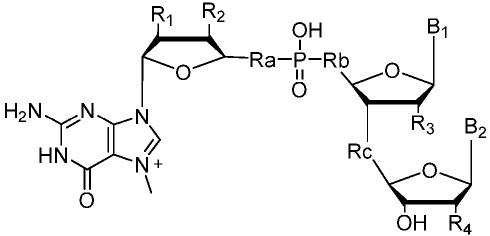
wherein R₁, R₂, R₃, and R₄ are independently hydrogen, halogen, hydroxyl, substituted or unsubstituted alkyl, substituted or unsubstituted O-alkyl, substituted or unsubstituted S-alkyl, substituted or unsubstituted NH-alkyl, substituted or unsubstituted N-dihydrocarbyl, substituted or unsubstituted O-aryl, substituted or unsubstituted S-aryl, substituted or unsubstituted NH-aryl, substituted or unsubstituted O-aralkyl, substituted or unsubstituted S-aralkyl, or substituted or unsubstituted NH-aralkyl;
when R₁ is substituted or unsubstituted O-alkyl, a bridged ring can be formed by connection of an alkyl carbon atom in the O-alkyl to a 4' carbon atom in a ribose group in which R₁ is located;
B₁ and B₂ are independently a natural, modified, or non-natural nucleobase;
Ra, Rb, and Rc independently have the following structure: 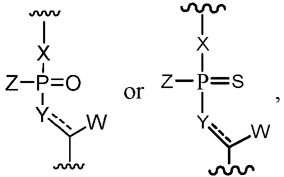 wherein Y is O, OCH₂, OCH, CH₂, CH, C₂H₃, or C₃H₅; Z is OH, SH, BH₃, aryl, alkyl, O-alkyl, or O-aryl; X is O, CH₂, or NH; W is H, OH, alkyl, O-alkyl, N-alkyl, S-alkyl, or halogen; -̅ -̅ -̅ represents a double bond or a single bond and is connected to a five-membered sugar ring;
and in at least one of the structures Ra, Rb and Rc, Y is O, OCH, or CH.

2. The vinylphosphonic acid-modified mRNA cap analog according to claim 1, wherein R₁, R₂, R₃, and R₄ are independently hydrogen, halogen, hydroxyl, substituted or unsubstituted alkyl, substituted or unsubstituted O-alkyl, substituted or unsubstituted S-alkyl, or substituted or unsubstituted NH-alkyl.

3. The vinylphosphonic acid-modified mRNA cap analog according to any one of claims 1-2, wherein the number of carbon atoms in the substituted or unsubstituted O-alkyl is 0-5.

4. The vinylphosphonic acid-modified mRNA cap analog according to any one of claims 1-2, wherein the number of carbon atoms in the substituted or unsubstituted alkyl is 0-5;
the number of carbon atoms in the substituted or unsubstituted S-alkyl is 0-5;
the number of carbon atoms in the substituted or unsubstituted NH-alkyl is 0-5;
the number of carbon atoms in the substituted or unsubstituted N-dihydrocarbyl is 0-5;
substituent groups in the substituted or unsubstituted alkyl, the substituted or unsubstituted O-alkyl, the substituted or unsubstituted S-alkyl, the substituted or unsubstituted NH-alkyl, and the substituted or unsubstituted N-dihydrocarbyl are independently selected from one or more of substituted or unsubstituted alkyl, substituted or unsubstituted O-alkyl, and substituted or unsubstituted acylamino.

5. The vinylphosphonic acid-modified mRNA cap analog according to claim 1, wherein B₁ and B₂ are independently adenine, guanine, cytosine, uracil, or thymine.

6. The vinylphosphonic acid-modified mRNA cap analog according to claim 1, wherein Ra, Rb, and Rc independently have the following structure: 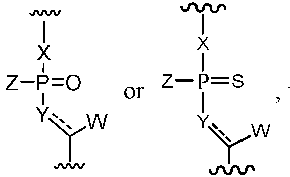 wherein Y is O, CH, C₂H₃, or C₃H₅; Z is OH or alkyl; X is O or CH₂; and W is H, OH, or alkyl;
and in at least one of the structures Ra, Rb and Rc, Y is CH.

7. The vinylphosphonic acid-modified mRNA cap analog according to claim 1, wherein R₁, R₂, and R₄ are all hydroxyl, and R₃ is methoxy; B₁ is adenine, and B₂ is guanine;
one or two of Ra, Rb, and Rc have the following structure:

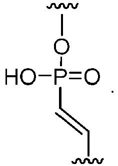

8. The vinylphosphonic acid-modified mRNA cap analog according to claim 1, wherein the vinylphosphonic acid-modified mRNA cap analog has any one of the following structures, or a pharmaceutically acceptable salt or stereoisomer thereof:

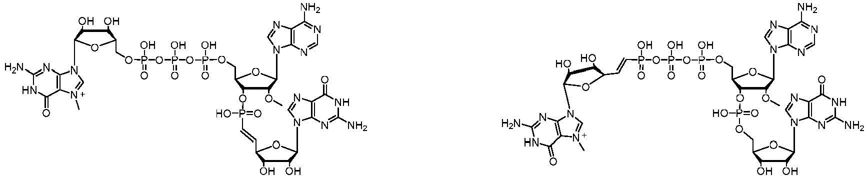

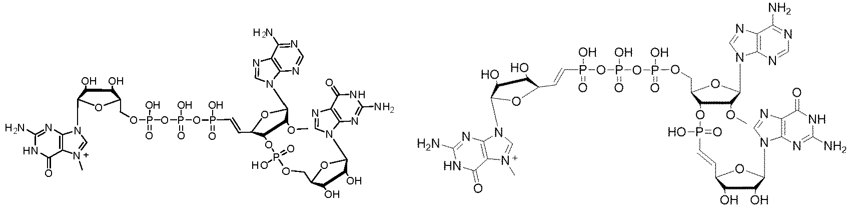

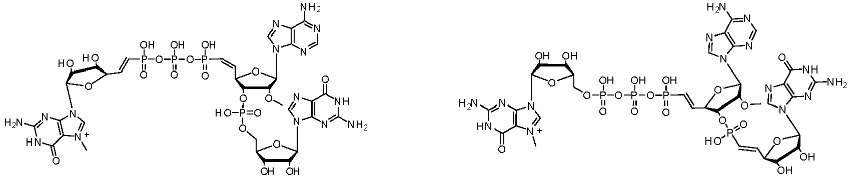

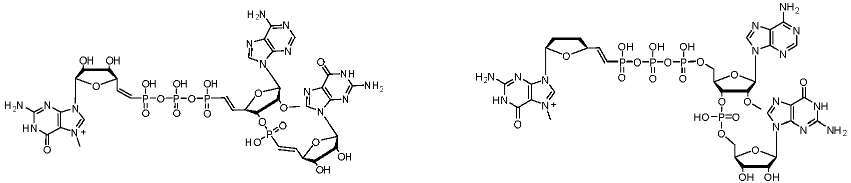

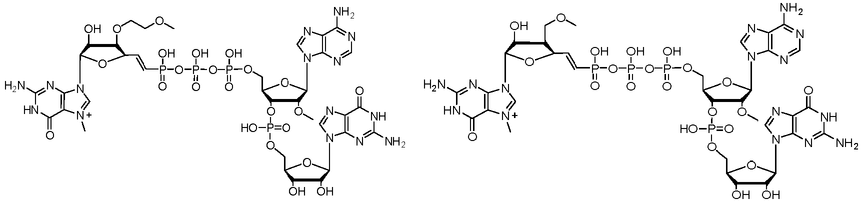

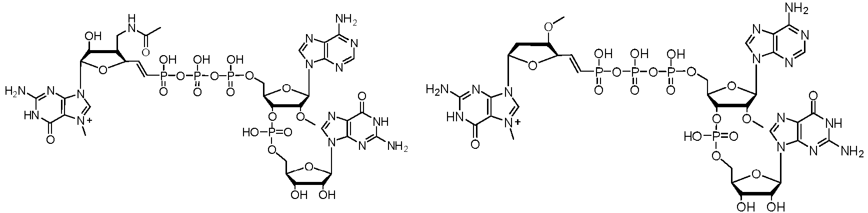

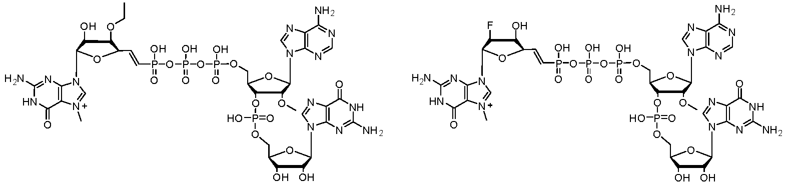

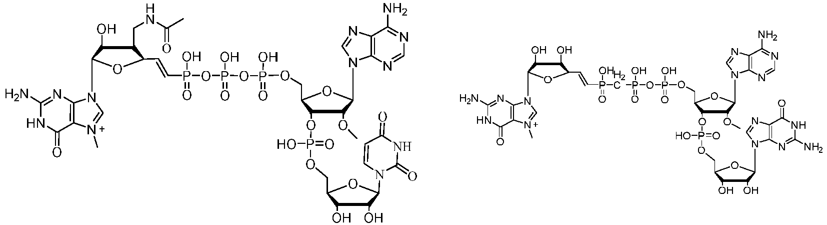

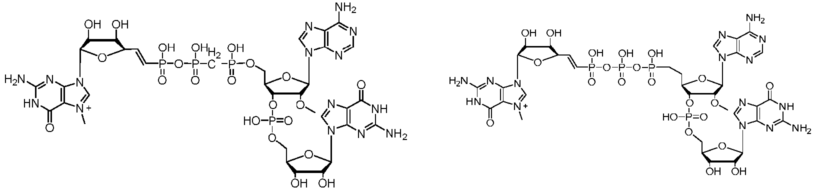

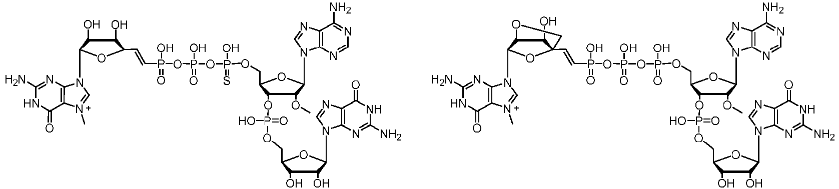

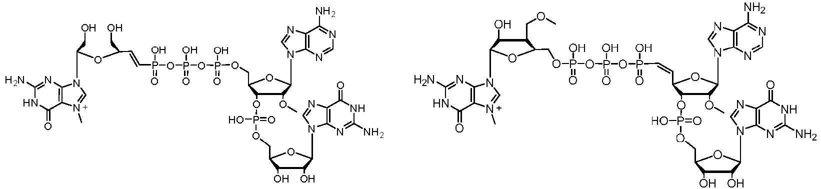

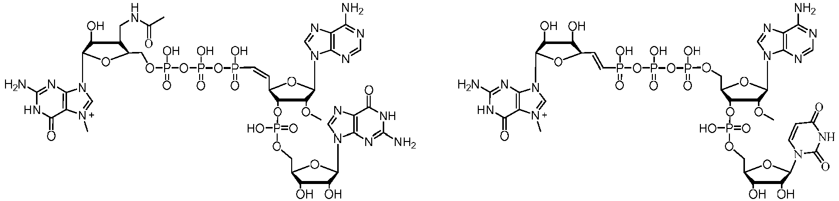

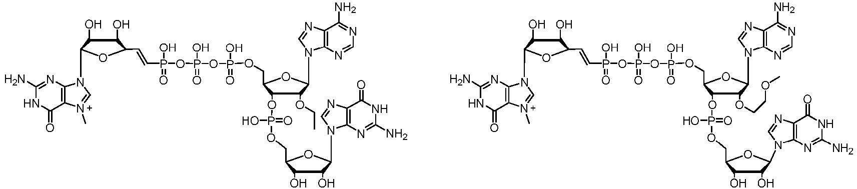

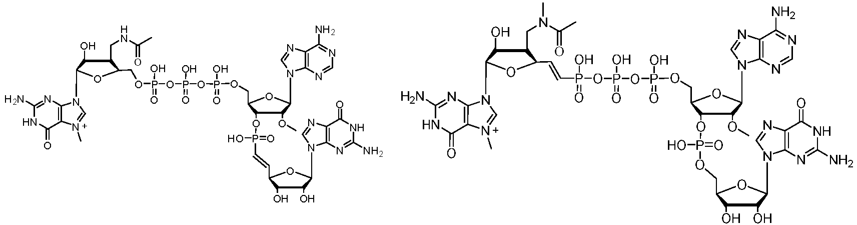

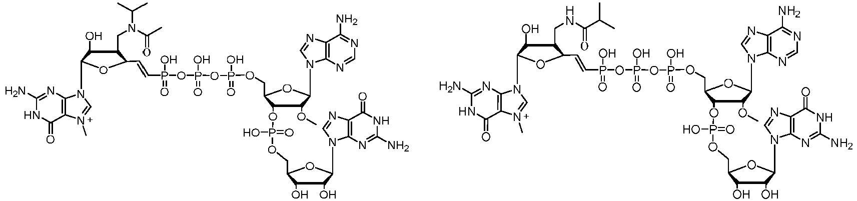

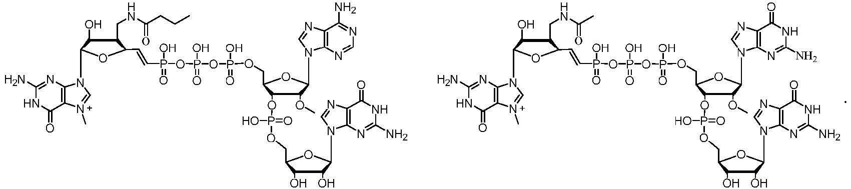

9. A method for preparing the vinylphosphonic acid-modified mRNA cap analog according to any one of claims 1-8, comprising the following steps: (1) synthesis of an imidazole salt of m7GDP or a modified analog thereof; (2) preparation of a phosphoester bond-linked dinucleotide; and (3) synthesis of the vinylphosphonic acid-modified mRNA cap analog.

10. The method for preparing the vinylphosphonic acid-modified mRNA cap analog according to claim 9, comprising the following steps: (1) the synthesis of the imidazole salt of m7GDP or the modified analog thereof: sequentially subjecting a guanosine or an analog thereof to diphosphorylation, methylation of N7, imidazolation reaction of diphosphoric acid, and the like to synthesize the imidazole salt of m7GDP or the modified analog thereof; (2) the preparation of the phosphoester bond-linked dinucleotide: coupling 2'OMe-A or a modified phosphoramidite monomer thereof with a protected guanosine or a modified analog thereof under the action of tetrazole to form a first phosphoester bond, removing a protecting group under the action of an acid, then introducing a second phosphoric acid, and finally performing hydrolysis to obtain the phosphoester bond-linked dinucleotide; and (3) the synthesis of the vinylphosphonic acid-modified mRNA cap analog: reacting the imidazole salt of m7GDP or the modified analog thereof with the phosphoester bond-linked dinucleotide to prepare the vinylphosphonic acid-modified mRNA cap analog.

11. Use of the vinylphosphonic acid-modified mRNA cap analog according to any one of claims 1-8, wherein the vinylphosphonic acid-modified mRNA cap analog is used for mRNA capping in a T7 RNA polymerase system.

12. A complex, comprising the vinylphosphonic acid-modified mRNA cap analog according to any one of claims 1-8 and a DNA template, wherein the DNA template comprises a promoter region comprising a transcription start site, the transcription start site having a first nucleotide at nucleotide position +1 and a second nucleotide at nucleotide position +2; the group B₁ in the structure of the vinylphosphonic acid-modified mRNA cap analog is complementary to a base of the first nucleotide, and the group B₂ in the structure of the vinylphosphonic acid-modified mRNA cap analog is complementary to a base of the second nucleotide.

13. An RNA molecule, comprising the vinylphosphonic acid-modified mRNA cap analog according to any one of claims 1-8.

14. A pharmaceutical composition, comprising the RNA molecule according to claim 13 and a pharmaceutically acceptable carrier.
